# EUROPEAN PATENT SPECIFICATION

(11) **EP 1 278 825 B1**
(45) Date of publication and mention of the grant of the patent: **14.06.2006**
(21) Application number: 01943246.7
(22) Date of filing: 11.04.2001
(51) Int. Cl.: C12N 5/06, C12N 5/08, A61K 39/00, G01N 33/53, C07K 14/765, C07K 14/745, C07K 16/00

(54) **METHODS OF PRODUCING MEMBRANE VESICLES**
VERFAHREN ZUR HERSTELLUNG VON MEMBRANVESIKELN
PROCEDES DE PRODUCTION DE VESICULES MEMBRANAIRES

(30) Priority: 27.04.2000 US 561205; 09.02.2001 US 780748
(43) Date of publication of application: 29.01.2003
(73) Proprietor: Exothera L.L.C., Menlo Park CA 94025 (US)
(72) Inventor: LAMPARSKI, Henry, San Mateo, CA 94402 (US); RUEGG, Curtis, Redwood City, CA 94062 (US); LE PECQ, Jean-Bernard, Menlo Park, CA 94025 (US); HSU, Di-Hwei, Sunnyvale, CA 94087 (US); YAO, Jenq-Yuan, Mountain View, CA 94040 (US)
(74) Representative: Becker, Philippe
(86) International application number: PCT/EP2001/004173
(87) International publication number: WO 2001/082958

(56) References cited:
- WO-A-00/28001
- WO-A-99/03499
- WOLFERS J ET AL: "Dendritic cell-derived exosomes (DEX) are effective cancer vaccines: Preclinical data towards a clinical trial." PROCEEDINGS OF THE AMERICAN ASSOCIATION FOR CANCER RESEARCH ANNUAL, no. 41, March 2000 (2000-03), page 874 XP002188693 91st Annual Meeting of the American Association for Cancer Research.;San Francisco, California, USA; April 01-05, 2000, March, 2000 ISSN: 0197-016X
- HSU DI-HWEI ET AL: "Dendritic cell (DC)-derived exosomes (DEX) activate T cells similar to DC: Implications for novel tumor vaccines." PROCEEDINGS OF THE AMERICAN ASSOCIATION FOR CANCER RESEARCH ANNUAL, no. 41, March 2000 (2000-03), page 875 XP002188694 91st Annual Meeting of the American Association for Cancer Research.;San Francisco, California, USA; April 01-05, 2000, March, 2000 ISSN: 0197-016X

## Description

### FIELD OF INVENTION

The present invention relates to methods of preparing biological material, for use in various experimental, diagnostic or therapeutic uses, including immunotherapy treatment or prophylaxy of tumors. More particularly, the present invention relates to methods of preparing membrane vesicles (in particular exosomes) released by various types of mammalian cells, comprising diafiltration and/or density cushion centrifugation. The invention also provides novel methods for characterizing and analysing exosome preparations, which can be used in quality control assay for the purpose of pharmaceutical product production, as well as methods of loading membrane vesicles with immunogenic compounds. The invention is suitable to produce pharmaceutical grade preparations of such membrane vesicles and to fully characterize said preparations, for use in human beings.

### BACKGROUND OF THE INVENTION

Membrane vesicles are essentially spherical vesicles, generally less than 130 nm in diameter, composed of a lipid bilayer containing a cytosolic fraction. Particular membrane vesicles are more specifically produced by cells, from intracellular compartments through fusion with the plasmic membrane of a cell, resulting in their release in biological fluids or in the supernatant of cells in culture. Such vesicles are generally referred to as exosomes. Exosomes are more particularly between about 30 and about 120 nm, preferably 50 and 90 nm, more specifically between about 60 and 80 nm in diameter and, advantageously, carry membrane proteins (particularly major histocompatibility complex proteins or other protein which directly or indirectly participate in antigen presentation). In addition, depending on their origin, exosomes comprise membrane proteins such as MHC I, MHC II, CD63, CD81 and/or HSP70 and have no endoplasmic reticulum or Golgi apparatus. Furthermore, exosomes are essentially void of nucleic acids (e.g. DNA or RNA).
Exosome release has been demonstrated from different cell types in varied physiological contexts. In particular, it has been demonstrated that B lymphocytes release exosomes carrying class II major histocompatibility complex molecules, which play a role in antigenic presentation (Raposo et al., J. Exp. Med. 183 (1996) 1161). Similarly, it has been demonstrated that dendritic cells produce exosomes (i.e., dexosomes, Dex), with specific structural and functional characteristics and playing a role in immune response mediation, particularly in cytotoxic T lymphocyte stimulation (Zitvogel et al., Nature Medicine 4 (1998) 594). It has also been demonstrated that tumor cells secrete specific exosomes (i.e., texosomes, Tex) in a regulated manner, carrying tumor antigens and capable of presenting these antigens or transmitting them to antigen presenting cells (patent application No. WO99/03499). It is also known that mastocyte cells accumulate molecules in intracellular vesicular compartments, which may be secreted under the effect of signals (Smith and Weis, Immunology Today 17 (1996) 60). Therefore, as a general rule, cells appear to emit signals and communicate with each other via membrane vesicles that they release, which may carry antigenic proteins (or polypeptides or peptides), MHC molecules or any other signal (cytokine, growth factor, etc.) with specific structural and functional characteristics, produced in different physiological situations. These vesicles, particularly exosomes, thus represent a product of particular interest for diagnostic, vaccination or therapeutic applications or to deliver molecules of interest. Therefore, it would be of particular interest to have an effective method that could be used at an industrial scale to prepare membrane vesicles compatible with biological use, particularly pharmacological use.

Conventional methods to prepare membrane vesicles (e.g. exosomes) involve a series of differential centrifugation steps to separate the vesicles from cells or cell debris present in the culture medium. In this regard, the documents mentioned above essentially describe the preparation of vesicles with a series of centrifugations at 300 g, 10,000 g and 70,000 g or 100,000 g, upon which the resulting pellet at the bottom of the tube is resuspended to 1/1000^{th} its original volume with a saline solution to constitute a concentrated exosome solution. However, these methods are essentially unsuitable for clinical applications for a number of reasons: 1) length of time, 2) scale-up and validation in GMP environment, 3) significant risk of contamination by cell debris, 4) poor reproducibility due to operator variability, 5) aggregation of exosomes resulting from pelleting (high localized exosome concentration in pellet) and 6) low recovery at end of processing. There is therefore a need for improved methods of preparing membrane vesicles, suitable with industrial constraints and allowing production of vesicle preparations of therapeutic quality.

International application n° PCT/FR00/00105 discloses methods of preparing membrane vesicles through chromatographic techniques, such as anion exchange chromatography and/or gel permeation chromatography. In International application n° WO-A-99 03499 and in documents "Proceedings of the American Association for Cancer Research" (2000, 41:874; 91^{st} Annual Meeting of the American Association for Cancer Research.; San Francisco, California, USA; April 01-05, 2000, Abstracts # 5556 and 5562), immunogenic membrane vesicles have been obtained by pulsing dendritic cells in culture.

### SUMMARY

The present invention now provides novel methods of preparing membrane vesicles in high yields, high purity, and in relatively short periods of time. The present description also discloses methods of characterizing (or analyzing or dosing) a membrane vesicle preparation, which can be used in pharmaceutical production to determine the activity, phenotype and/or quantity of vesicles. The invention now allows the production and characterization of clinically acceptable lots of membrane vesicles, with reproducibility, limited operator variation, and increased product quality. Also discloses are methods of removing particulate bodies, such as haptoglobin, from various medium or compositions, the resulting compositions and media and their uses. This invention also concerns novel methods of loading membrane vesicles with immunogenic compounds and uses thereof.

More specifically, an aspect of the present invention resides in methods of preparing membrane vesicles using density cushion centrifugation.

Another aspect of the present invention resides in methods of preparing membrane vesicles using a series of ultrafiltration steps and/or clarification step, more specifically a combination of a concentration and diafiltration by ultrafiltration, preferably preceded by a clarification.

Another aspect of this invention resides in methods of preparing membrane vesicles using a combination of density cushion centrifugation and ultrafiltration and/or clarification step, more specifically a combination of a concentration and diafiltration by ultrafiltration, preferably preceded by a clarification, followed by density cushion centrifugation.

In a particular aspect, the method of this invention comprises a density cushion centrifugation preceded or followed by a diafiltration.

The method of this invention can be applied to various biological samples containing membrane vesicles, including a biological fluid, a culture supernatant, a cell lysate or a pre-purified solution. In a particular embodiment, the method is used to prepare (e.g., purify or separate or isolate) membrane vesicles from a biological sample enriched with membrane vesicles.

Also disclosed is a method of preparing membrane vesicles from a biological sample, comprising :
a. the culture of a population of membrane vesicle-producing cells under conditions allowing the release of the vesicles,
b. a membrane vesicle enrichment step, and
c. the treatment of said enriched biological sample by density cushion centrifugation.

The membrane vesicle-producing cells are cultured in a culture medium with reduced particulate bodies' content, preferably a medium deprived of haptoglobin aggregates. As will be demonstrated in this application, the use of such a medium allows increased production yields and/or higher purity and quality levels to be achieved.

The enrichment step may comprise one or several centrifugation, clarification, ultrafiltration, nanofiltration, affinity chromatography and/or diafiltration steps. More preferably, the enrichment step comprises a clarification and/or a concentration and/or a diafiltration.

The preparation of exosomes may be collected from step c) by any appropriate means, including pipetting or with a needle.

The method may further comprises a sterile filtration d) of the preparation from step c.

The present invention can be used to prepare membrane vesicles from various origins, including membrane vesicles produced by antigen-presenting cells (such as macrophages, dendritic cells, B lymphocytes), tumor cells or any other cell or cell line producing vesicles, preferably transduced for antigens. It is particularly suited for preparing membrane vesicles produced by dendritic cells, preferably immature dendritic cells (i.e., dexosomes). Furthermore, the membrane vesicles or corresponding producing cells can be sensitized to one or several antigens, prior to, during or after preparation.

Methods disclosed comprise:
- a method of preparing membrane vesicles, comprising :
   b. the culture of a population of antigen-presenting cells, in particular dendritic cells, under conditions allowing the release of membrane vesicles by antigen-presenting cells, in particular dendritic cells,
   c. a membrane vesicle enrichment step, and
   d. the isolation of the membrane vesicles using density cushion centrifugation.
- a method of preparing membrane vesicles, comprising :
   a. obtaining a population of immature dendritic cells
   b. culturing the population of immature dendritic cells under conditions allowing the release of membrane vesicles by immature dendritic cells,
   c. a membrane vesicle enrichment step, and
   d. the isolation of the membrane vesicles using density cushion centrifugation.

As indicated above, an additional step of sensitization of the vesicles (or producing cells) to one or several particular antigens can be introduced in the process, either before step b), to sensitize the producing cells, or after step b), to sensitize directly the membrane vesicles.

In this regard, the present invention discloses a procedure for direct loading of exosomes, in particular for direct incorporation of immunogenic compounds (such as peptides or lipids) into MHC complexes on exosomes previously prepared and purified from antigen-presenting cell cultures. The method according to this invention is particularly advantageous for direct loading of peptides into MHC (class I) complexes on dexosomes previously purified from immature dendritic cell cultures.
As indicated above; loading of exosomes derived from dendritic cells (dexosomes) with HLA class I or II associated peptides has up to now been approached by indirect loading, i.e., by adding a therapeutic (antigenic) peptide (e.g., class I restricted tumor peptide) (e.g., at 10 µg/ml on day 6) to the culture of vesicle producing cells (e.g., dendritic cells (DC) differentiated from monocyte-enriched adherent cells in the presence of GM-CSF and IL-4) (see for instance US5,846,827 and cited references). It is assumed that the DC take up the exogenous peptide and thereby produce dexosomes harboring these class I peptides on the exosome surface. *In vivo* experiments with the murine P1A mastocytoma tumor model show that dexosomes produced in this manner elicit antitumor immunological responses that resulted in tumor regression. Dexosomes indirectly loaded with Mart-1 peptide could also stimulate marginal secretion of IFN-γ from a Mart-1 specific CTL clone, LT 11, suggesting that some degree of peptide loading did occur. However, even using time resolved fluorometry (TRF), a biochemical approach with sensitivity similar to radioactive detection, binding of class I peptide on these dexosomes was hardly detectable.
In order to improve the efficacy and industrial implementation of the process, the inventors have now surprisingly found that it is possible to perform direct loading approach, in which the step of adding peptide to DC culture is replaced by adding the peptide onto dexosomes after they are purified from DC culture. In particular, the inventors have now discovered that selected acid media or buffer can be developed and defined to protect membrane vesicles and allow the direct loading of immunogenic compounds to antigen-presenting molecules at the surface of said vesicles, particularly to MHC molecules such as Class-I, Class-II or CD1 molecules.
A particular object of the present invention thus also resides in a method of preparing an immunogenic membrane vesicle, the method comprising isolating or purifying the membrane vesicle from a biological sample and contacting said isolated or purified membrane vesicle with an immunogenic compound (e.g., a peptide or lipid) under conditions allowing the immunogenic compound to bind an antigen-presenting molecule (e.g. MHC class I or class II for peptides and CD1 for lipid antigens) at the surface of said membrane vesicle.
In a preferred embodiment, the method comprises the step of subjecting the isolated or purified membrane vesicles to a selected acid medium or treatment prior to, during, or after contacting said vesicles with said immunogenic compound, so as to enable or facilitate loading thereof. In this regard, the use of selected acid media or treatments as disclosed in the present application allows to at least partially remove endogenous peptides or lipids associated at the surface of the vesicles and/or to facilitate the exchange of immunogenic compounds.
In a further preferred embodiment, after contacting with the immunogenic compound, the vesicles are subjected to centrifugation, preferably density centrifugation, or diafiltration, to remove unbound immunogenic compound.

The immunogenic compound may be for instance any peptide or lipid, which are presented to an immune system in association with antigen-presenting molecules. The peptides may be class-I restricted peptides, class-II restricted peptides, either alone or in mixture or combination with other peptides, or even a peptide eluate of tumor cells. The invention is particularly suited for direct loading of class-I-restricted peptides. The lipids may be a microbial lipid, a microbial glycolipid or a lipid or glycolipid tumor antigen, either in isolated form or in various combination(s) or mixture(s).

The direct loading method provides significant advantages over prior techniques. In particular, as illustrated int the examples, the direct peptide loading is more efficient than prior indirect loading, in that a higher occupancy rate of surface HLA receptors can be obtained using lower amounts of peptide. This clearly increases the immunogenic potential of the membrane vesicles. In addition, the structure of the loaded peptide can be controlled more precisely, since the loaded peptides are not processed by whole cells and thus remain intact and un-modified upon loading. The invention now shows for the first time that direct peptide loading can be carried out using purified membrane vesicles such as dexosomes. In this regard, the invention shows that membrane vesicles stand low pH conditions without loosing their activity and functionality. The invention also describes particular conditions allowing improved direct loading. The invention particularly show that conventional buffer media used for indirect loading (i.e., loading of whole cells) may advantageously be replaced with different buffer media to increase the direct loading efficiency and obtain higher occupancy rates, indicating that vesicles do not behave as whole cells.

In a preferred embodiment, the invention is directed to a method for preparing an immunogenic membrane vesicle comprising a complex of an exogenous class-I peptide bound to an HLA class I molecule, the method comprising (i) subjecting an isolated or purified membrane vesicle to a selected acid medium, (ii) contacting said isolated or purified membrane vesicle with a class I-restricted peptide under conditions allowing the peptide to complex with an HLA class I molecule at the surface of said membrane vesicle, and (iii) collecting the loaded membrane vesicle. The term "exogenous" means that the peptide is added to the composition.

In a particular variant, the method comprises the steps of (i) subjecting an isolated or purified membrane vesicle to selected acid medium, (ii) contacting said isolated or purified membrane vesicle with a class I-restricted peptide in the presence of beta2-microglobulin, under conditions allowing the peptide to complex with an HLA class I molecule at the surface of said membrane vesicle, and (iii) collecting the loaded membrane vesicle. More preferably, step (i) comprises subjecting the isolated or purified membrane vesicles to an acid medium at a pH comprised between about 3 and 5.5, even more preferably between about 3.2 and 4.2, for less than 5 minutes. The direct loading approach in the presence of beta2-microglobulin is advantageous since it allows efficient loading even where only very small amounts of immunogenic compounds are available. Indeed, when beta2-microglobulin is used, the inventors have shown that it is possible to use defined acid media that remove essentially all endogenous peptides from membrane vesicles and thus favor reconstitution of functional MHC complexes even with low levels of immunogenic compounds (e.g., from 0.005 to 50 µg/ml). This approach is thus particularly suited for direct loading of tumor eluates, lipids, natural antigens or other immunogenic compounds that cannot be produced economically in high quantities (e.g., by synthesis).

Where higher amounts of immunogenic compounds are available, the inventors have defined a second general direct loading approach, in which no beta2-microglobulin is required. This embodiment is advantageous since less treatments are needed (so that the exosomes are not altered) and there is no need for exogenous β2-m, which can reduce the costs and potential drawbacks of adding exogenous material to the process. In this respect, in an other particular variant, the method comprises the steps of (i) contacting an isolated or purified membrane vesicle with a class I-restricted immunogenic compound (e.g., peptide or lipid) in the absence of beta2-microglobulin, (ii) subjecting the mixture of (i) to a selected acid medium or treatment under conditions allowing the immunogenic compound to exchange with any endogenous compound for binding with an HLA class I molecule at the surface of said membrane vesicle, (iii) neutralizing the medium to stop the exchange and/or stabilize the complex formed in (ii) and (iv) collecting the loaded membrane vesicle. More preferably, step (ii) comprises subjecting the mixture to an acid medium at a pH comprised between about 4 and 5.5 for a period of time sufficient to produce an exchange between any endogenous molecule and the immunogenic compounds, for binding to the MHC complex. Indeed, the inventors have now shown that, in contrast with whole cells, membrane vesicles may resist prolonged exposure to acid medium or conditions, thus allowing replacement of endogenous molecules by any desired immunogenic compound, without altering or destabilizing the MHC molecule and essentially without degrading or removing endogenous β2-m. The inventors have also defined particular acid media and conditions allowing the exchange to be performed without altering the functionality of membrane vesicles. These selected media are more particularly characterized by a pH comprised between about 4 and 5.5, more preferably between 4.2 and 5.2. The membrane vesicles are preferably contacted with the above selected acid media for a period of time above 15 minutes, typically less than 2 hours, even more preferably for less than 1 hour. According to this variant, the method does not comprise the use of beta2-microglobulin and loading occurs through an exchange between the desired peptide and the endogenous peptide, without releasing endogenous β2-m. In this beta2-microglobulin-free embodiment, the isolated or purified membrane vesicle is typically contacted with an excess of exogenous immunogenic compound, e.g., with 5 to 500 µg/ml of class I-restricted peptide or lipid, in the absence of beta2-microglobulin. This embodiment is particularly suited for loading immunogenic compounds available in large quantities, such as peptides produced by synthesis.

As indicated above, the method can be performed using various immunogenic molecules. The peptides are preferably peptides presented by class I or Class II molecules, i.e., peptides that result from processing of antigens by antigen presenting cells such as dendritic cells and macrophages and B lymphocytes. A preferred class of peptides is represented by class-I restricted peptides, even more preferably class-I restricted tumor peptides, i.e., peptides derived from tumor antigens, which are presented by dendritic cells or macrophages to the immune system. Specific peptides include for instance HLA-A1, HLA-A2, HLA-A3, HLA-A24, HLA-B35 or other haplotypes-restricted tumor peptides, from various tumor cells or antigens, such as Mage, Bage, Mart, CEA, PSA, etc. It should be understood that the method can be performed using isolated peptides or mixtures thereof, such as peptide eluates of tumor cells or other peptide combinations. Antigenic peptides derived from other pathogenic agents (viruses, cells, parasites, etc.) can also be loaded according to the present invention, so as to produce immunogenic membranes vesicles suitable for treating various disease conditions such as cancers, infectious, or immune diseases, for instance.

The lipids may be any lipid, glycolipid or lipoprotein presented to an immune system by CD1 molecules at the surface of antigen-presenting cells. CD1 molecules are MHC-like molecules responsible for presenting lipid or glycolipid antigens to T cells. Several isotypes of CD1 have been described, including CD1a, CD1b, CD1c, CD1d and CD1e, which are involved in presentation of lipid antigens. In a particular embodiment, the method is used to load lipid antigens to CD1 molecules at the surface of membrane vesicles, particularly to CD1b and CD1d. The lipid may be any microbial lipid, microbial glycolipid, lipid or glycolipid tumor antigen, etc. More preferred examples of lipids include mycobacterial lipids such as mycolic acids, glucose monomycolate, hexose-1-phosphoisoprenoids and derivatives of lipoarabinomannans (LAM) such as phosphatidylinositolmannosides. Other examples include self ceramides such as gangliosides, glycolipid tumor antigens, etc.

Particular methods comprise:
- a method of preparing immunogenic membrane vesicles, comprising :
   b) the culture of a population of antigen-presenting cells, in particular dendritic cells, under conditions allowing the release of membrane vesicles by antigen-presenting cells, in particular dendritic cells,
   c) a membrane vesicle enrichment or purification step, and
   d) the contacting of the membrane vesicles with an immunogenic compound under conditions allowing the immunogenic compound to bind an MHC molecule at the surface of said membrane vesicles to produce immunogenic membrane vesicles.
- a method of preparing immunogenic membrane vesicles, comprising:
   a. obtaining a population of immature dendritic cells
   b. culturing the population of immature dendritic cells under conditions allowing the release of membrane vesicles by immature dendritic cells,
   c. a membrane vesicle enrichment or purification step, and
   d. the contacting of the membrane vesicles with an immunogenic compound under conditions allowing the immunogenic compound to bind an MHC molecule at the surface of said membrane vesicles to produce immunogenic membrane vesicles.
- a method of preparing immunogenic membrane vesicles, comprising:
   a. obtaining a population of immature dendritic cells
   b. culturing the population of immature dendritic cells under conditions allowing the release of membrane vesicles by immature dendritic cells,
   c. a membrane vesicle enrichment or purification step, and
   d. the contacting of the membrane vesicles with a lipid antigen under conditions allowing the lipid antigen to bind a CD 1 molecule at the surface of said membrane vesicles to produce immunogenic membrane vesicles.

Enrichment or purification are preferably performed as described above, as well as direct loading.

A particular method resides in a method of preparing membrane vesicles, comprising:
a. obtaining a population of antigen-presenting cells, more preferably immature dendritic cells,
b. optionally, sensitizing the antigen-presenting cells, more preferably the immature dendritic cells to one or several antigens,
c. culturing the population of antigen-presenting cells, more preferably immature dendritic cells under conditions allowing the release of membrane vesicles by antigen-presenting cells, more preferably immature dendritic cells,
d. a clarification of the culture supernatant,
e. a concentration of the clarified supernatant,
f. a diafiltration of the concentrated supernatant,
g. the isolation of the membrane vesicles using density cushion centrifugation,
h. optionally, the contacting of the membrane vesicles with a peptide to produce peptide-loaded membrane vesicles, and
i. a sterile filtration of the membrane vesicles obtained in g.

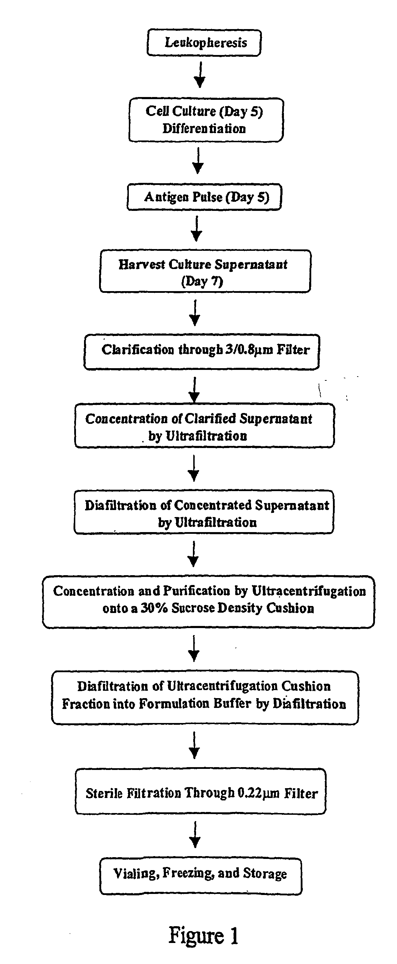

Sterile filtration i) may be preceded by a buffer exchange step, for instance through diafiltration. A typical process scheme is depicted on Figure 1. In the above process, either step b) or step h) are performed.

Furthermore, the present disclosure also provides methods of removing particulate bodies from various media or compositions. More particularly, it is demonstrated that conventional culture media contain particulate bodies, such as haptoglobin and related polymers (e.g., haptoglobin aggregates), and discloses methods of removing the same. More generally, the methods allow the production of culture media or any other biological products, such as blood proteins or polypeptides (or derivatives thereof), formulation solutions, fetal calf serum, etc., that are essentially deprived of haptoglobin aggregates.

Haptoglobin aggregates can exhibit immunosuppressive activity and thus affect the biological properties, safety and purity of membrane vesicles or other biological products, as will be further documented below. However, the issue of particular bodies was not addressed in the art, their presence in various biological products not determined, and efficient methods of removing the same not available. The invention now provides efficient methods to prepare high quality biological products, said resulting products also representing objects of this invention.

Another aspect of the present disclosure resides in method of producing or culturing antigen-presenting cells (or any membrane vesicle-producing cell), in particular dendritic cells, using culture or production media with reduced particulate bodies content. More preferably, the culture medium is a culture medium that is essentially free of haptoglobin aggregates, more specifically treated by ultrafiltration.

The invention is also suitable for the production of compositions of blood products that are essentially free of aggregated haptoglobin, as well as to the treatment of various buffer solutions prior to formulating products for pharmaceutical uses.

The disclosure also resides in a method of treating a biological product, more preferably a heat inactivated biological product, in order to reduce the amount of haptoglobin aggregates contained therein, comprising subjecting the product to filtration, more preferably ultrafiltration.

A particular object of this disclosure also resides in a method of preparing a biological product comprising (i) a heat inactivation of the biological product and (ii) a filtration of the heat inactivated biological product. More preferably, the method further comprises the step of (iii) concentrating the filtered, heat inactivated biological product and/or (iv) the conditioning thereof. The method can be used for various biological products including any protein or polypeptide (or derivatives thereof) isolated (or extracted) from mammalian biological fluids such as human blood or plasma or serum. As will be further documented in this application, this method allows, for the first time, the production of heat inactivated biological products having a reduced content in haptoglobin aggregates, more preferably essentially free of haptoglobin aggregates and thus with increased safety. The method is particularly suited for the preparation of pharmaceutical proteins extracted from blood or plasma such as serum-albumin, more preferably a human serum-albumin, gamma immunoglobulin, coagulation factors, etc.

The disclosure also comprises in a method of treating a serum preparation, more preferably a fetal calf serum preparation, to reduce the amount of haptoglobin aggregates contained therein, comprising subjecting the preparation to filtration, more preferably ultrafiltration.

As will be further documented below, the expression "essentially free" indicates that the composition or medium contains less than about 1 ppm of haptoglobin aggregates, more preferably less than about 0.5 ppm, even more preferably no detectable haptoglobin aggregates by SDS PAGE analysis as well as by quantitative ELISA.

Another aspect of this disclosure resides in methods of analyzing or characterizing (or dosing) membrane vesicles in a preparation, in order to determine their phenotype and/or activity and/or quantity.

More particularly, an aspect of this disclosure lies in a method of characterizing membrane vesicles, comprising contacting the membrane vesicles in parallel with two or more antibodies specific for marker components of membrane vesicles and determining the formation of antigen-antibody immune complexes.

Another particular disclosure comprises a method of characterizing the activity of a preparation of membrane vesicles, comprising contacting super-antigen-loaded vesicles with T cells in the presence of accessory cells, and determining the activation of the T cells.

The disclosure also provides a method of dosing membrane vesicles in a sample, comprising (i) loading the sample onto a solid support, (ii) contacting the support with an anti-class II antibody (or other relevant antibodies) and, (iii) determining the presence of antibody-antigen immune complexes.

The disclosure also encompasses methods of preparing a pharmaceutical product comprising an immunogenic membrane vesicle and a pharmaceutically acceptable diluent or carrier, wherein the method comprises (i) isolating a membrane vesicle from a biological sample (e.g., containing antigen-presenting cells (e.g. dendritic cells)), (ii) loading said isolated membrane vesicle with an immunogenic compound to produce an immunogenic membrane vesicle, (iii) preferably, removing unbound immunogenic compound (advantageously by density centrifugation or diafiltration), and (iv) contacting the immunogenic membrane vesicle with a pharmaceutically acceptable diluent or carrier.

A method of producing an immune response in a subject comprising (i) obtaining a biological sample containing dendritic cells, (ii) isolating or purifying a membrane vesicle from said biological sample, (iii) contacting said purified membrane vesicle with an immunogenic compound under conditions allowing the immunogenic compound to bind an MHC molecule at the surface of said membrane vesicle, and (iv) administering the membrane vesicle of (iii) to the subject to produce an immune response in said subject is also herein described. The subject is preferably a human being, although veterinary uses are also contemplated.

The methods of this disclosure particularly suited for preparing dexosomes (i.e., membrane vesicles produced by dendritic cells) or texosomes (i.e., membrane vesicles produced by tumor cells), more particularly of human origin. These membrane vesicles can be used in various experimental, biological, therapeutic, diagnostic or prophylactic applications. In particular, the membrane vesicles can be used to modulate an immune response in a subject, in particular in pathological conditions such as cancers, auto-immune diseases, allergy, asthma, inflammation and the like.

### LEGEND TO THE FIGURES

**Figure 1**. Overview of Particular Process for Autologous Dexosome Isolation and Purification.

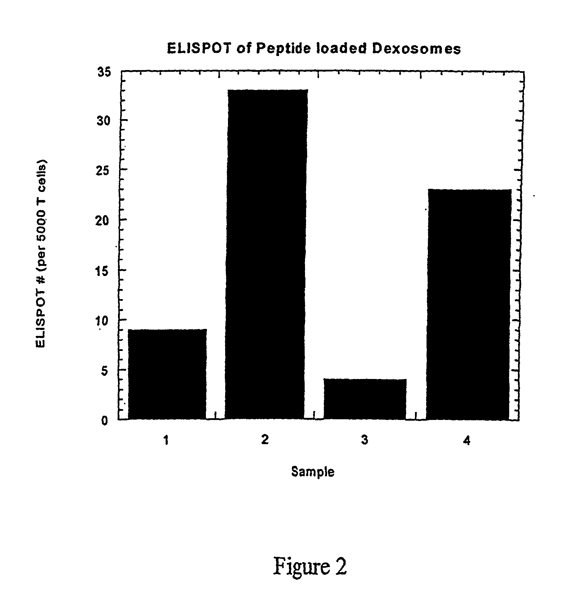

**Figure 2**. DC were cultured in the presence of CMV pp65 HLA-A2 restricted peptide on Day 5. Dexosomes were harvested on Day 7 and purified. Purified CMV peptide-pulsed dexosomes were then added to a CMV pp65 HLA-A2 restricted T cell line in the presence of HLA-A2 positive or HLA-A2 negative DC. Cell cultures were conducted in ELISPOT format where culture wells were coated with antibody specific for IFN-γ and the presence of cells specifically secreting this cytokine were enumerated as a measure of T cell activation.

Samples are as follows:
1. T + A2⁺ DC + exo/A2⁺ DC/ No peptide
2. T + A2⁺ DC + exo/A2⁺ DC/ CMV peptide
3. T + A2⁻ DC + exo/A2⁺ DC/ No peptide
4. T + A2⁻ DC + exo/A2⁺ DC/ CMV peptide

The specific peptide response of T cells to Dexosomes can be viewed by comparing sample 2 to sample 1 and proof that the stimulation is due to peptide incorporated in Dexosomes and not due to free peptide stimulating DC directly is illustrated by comparing sample 4 to sample 3.

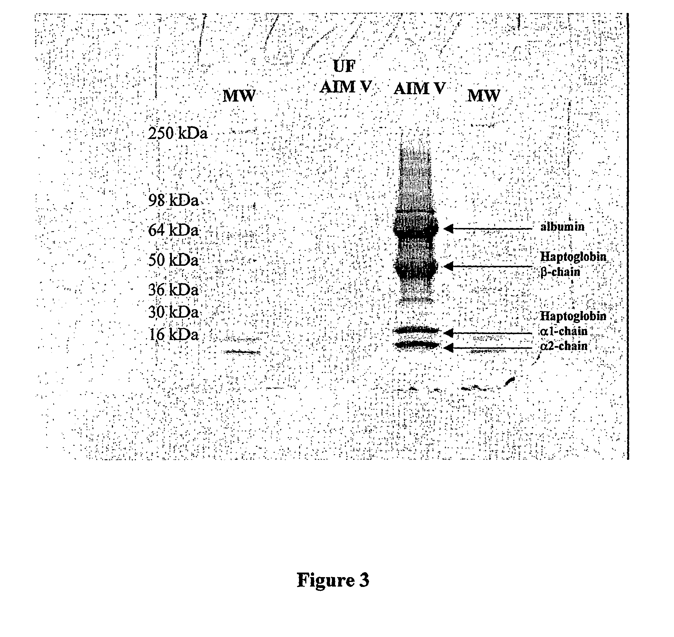

**Figure 3**. SDS-PAGE of AIM V media before and after processing by ultrafiltration through 500kDa (MWCO) hollow fiber membrane. AIM V and UF-processed AIM V were pelleted by ultracentrifugation at 100,000 x g for 1 hour. The supernatant was discarded and the pellet region was resuspended in PBS and re-ultracentrifuged a 2^{nd} time at 100,000 x g for 1 hour. The pellet was resuspended to 1/1000^{th} the original volume with PBS. 20uL of the pellet (reducing conditions) was run on an 8-16% acrylamide gel that was subsequently stained with colloidal Coomassie Blue.

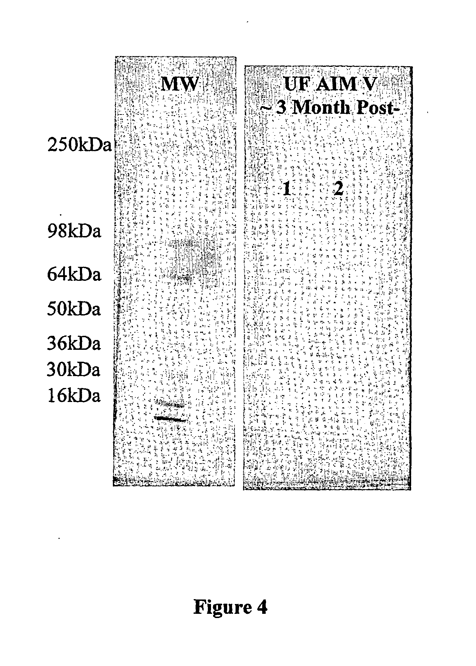

**Figure 4**. SDS-PAGE of AIM V media 3 months post-processing by ultrafiltration through 500kDa (MWCO) hollow fiber membrane. UF-processed AIM V was pelleted by ultracentrifugation at 100,000 x g for 1 hour. The supernatant was discarded and the pellet region was resuspended in PBS and re-ultracentrifuged a 2^{nd} time at 100,000 x g for 1 hour. The pellet was resuspended at 1/1000^{th} the original volume with PBS. 20uL of the pellet (reducing conditions) was run on an 8-16% acrylamide gel that was subsequently stained with colloidal Coomassie Blue.

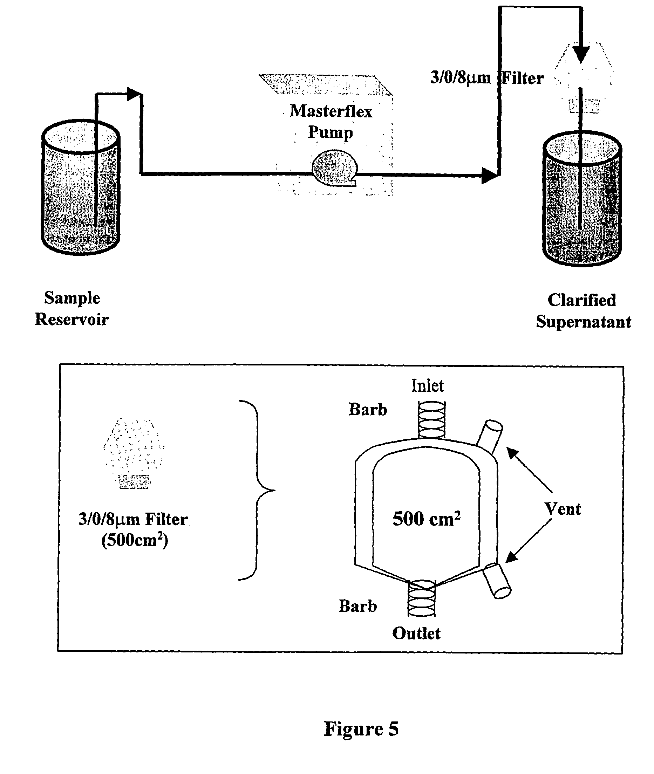

**Figure 5.** Schematic of the microfiltration system with 3/0.8 µm capsule filter

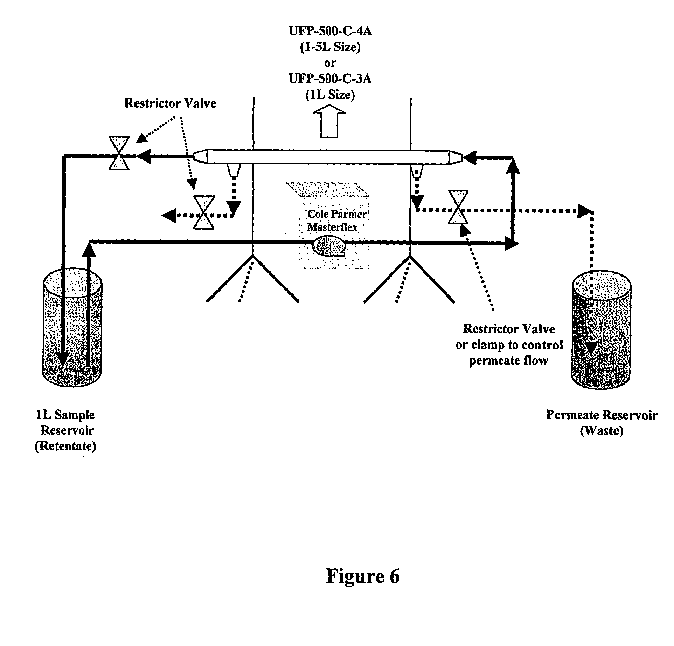

**Figure 6**. Scheme: Ultrafiltration of Clarified Tissue Culture Supernatant Using a Hollow Fiber Cartridge System.

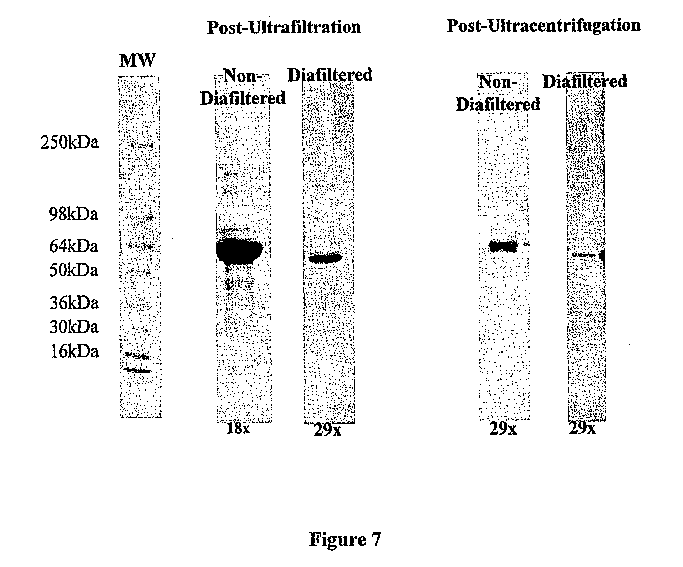

**Figure 7**. SDS-PAGE of a concentrated tissue culture supernatant containing dexosomes prior to and after diafiltration with PBS with a 500kDa hollow fiber membrane. The sample was diafiltered with 5 volumes of PBS. Pre- and post-diafiltered dexosomes were further purified by ultracentrifugation onto a density cushion composed of 30% sucrose/Tris D20 buffer. The fold concentration is depicted for each sample analyzed. 20uL of the pellet (reducing conditions) was run on an 8-16% acrylamide gel that was subsequently stained with colloidal Coomassie Blue.

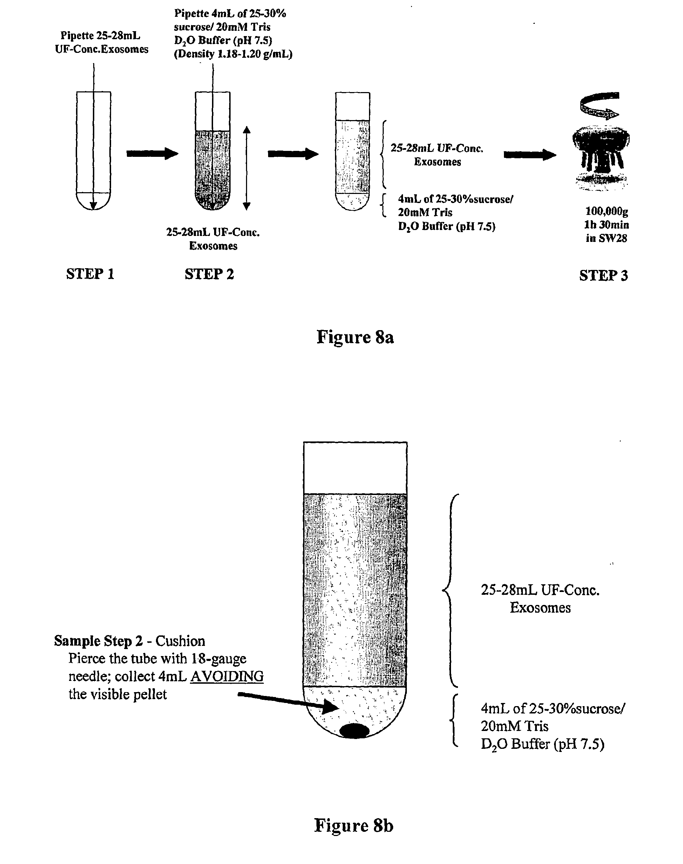

**Figure 8a.** Ultracentrifugation of concentrated Dexosomes into a 30% sucrose/Tris D₂O density cushion (density = 1.190-1.210g/mL)- Sample Preparation
**Figure 8b**. Sample collection from Dexosomes ultracentrifuged onto a density cushion composed of 30% sucrose/Tris D₂O buffer. The pellet is resuspended to 1/1000^{th} of its original volume with formulation buffer.

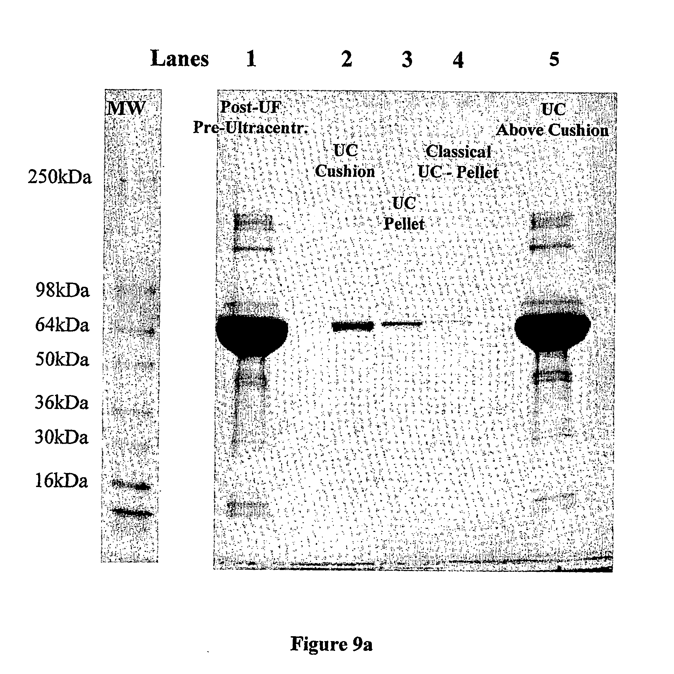

**Figure 9a**. SDS-PAGE of dexosomes fraction collected after ultracentrifugation onto a density cushion composed of 30% sucrose/Tris D2O buffer. All samples were normalized to 1/21th the original volume. Fractions were as follows: Lane 1Post-Ultrafiltration/Pre-Ultracentrifugation, Lane 2 - UC density cushion fraction, Lane 3 - UC pellet at bottom of cushion / reconstituted to 1/1000^{th} original volume, Lane 4 - UC pellet obtained classical sedimentation method / reconstituted to 1/1000^{th} original volume, and Lane 5 - UC above cushion

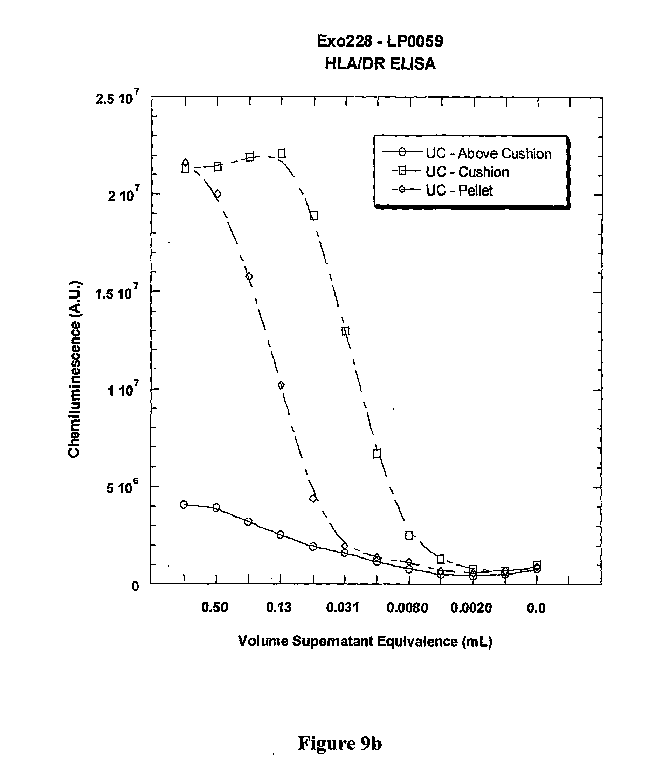

**Figure 9b.** HLA/DR ELISA of different fractions of the sucrose/D2O density cushion after ultracentrifugation at 100,000 x g for 1 hour. No HLA/DR is observed above the cushion, approximately 10% of the signal in the reconstituted pellet (1/1000^{th}), and 90% in the cushion.

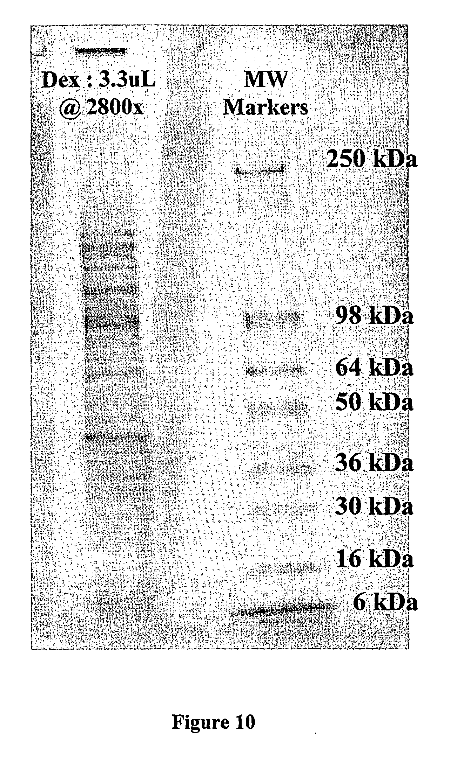

**Figure 10.** SDS-PAGE of dexosomes and diafiltration into PBS formulation buffer. Dexosomes were concentrated further by sedimentation by ultracentrifugation at 100,000 x g for 1hour. The pellet was reconstituted in PBS to 1/2800^{th} the starting supernatant volume. 3.3uL of dexosomes (reducing conditions) was loaded onto a 8-16% acrylamide gel that was subsequently stained with colloidal Coomassie Blue.

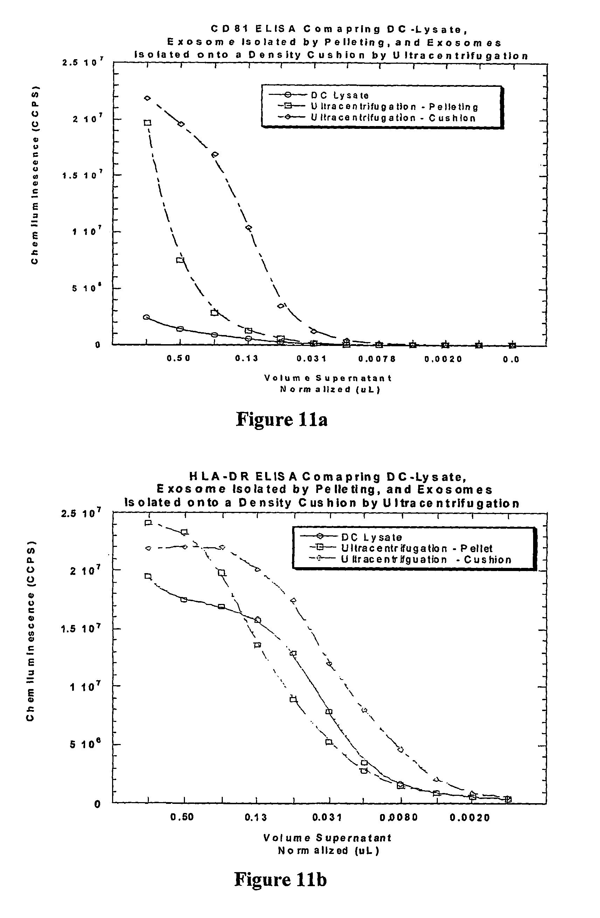

**Figure 11.** ELISA of Exosomes. Exosomes were purified by either pelleting by ultracentrifugation or by ultracentrifugation onto a density cushion solution (30% sucrose/20mM Tris in D20). For comparison, DC lysate was used at equivalent cell culture equivalents. Non-specific binding sites were blocked with nonfat dry milk and CD81 (figure 11a) and HLA-DR (figure 11b) were detected via specific mAb followed by detection with horseradish peroxidase conjugated secondary antibodies. Samples were quantitated by chemiluminescent detection and plotted as a function of cell culture equivalence.

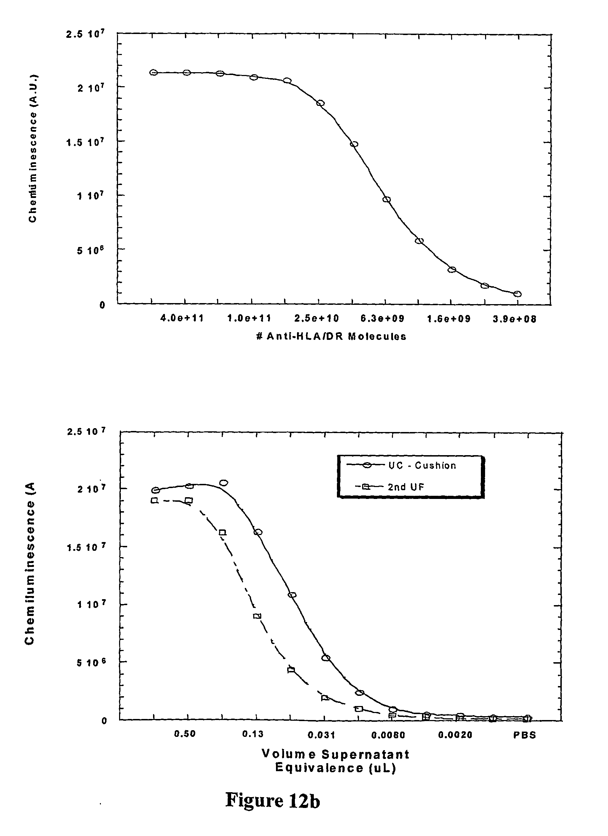

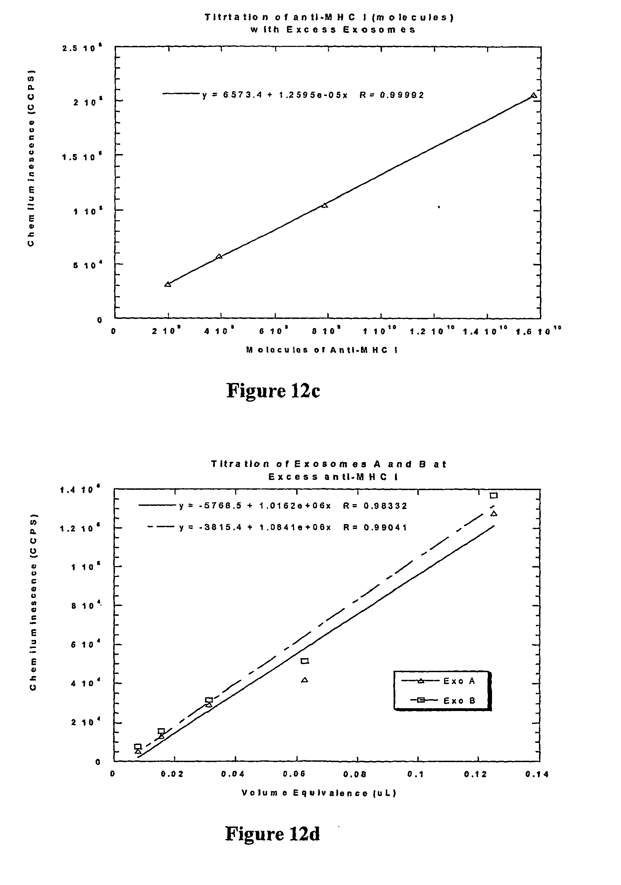

**Figure 12.** HLA/DR (Figure 12a,b) and MHC I (Figure 12c,d) ELISA of a Dexosome Preparation. Dexosome preparation was made by the methods described previously. Cell culture supernantant (3.7L) was clarified by filtration, concentrated by ultrafiltration, concentrated by ultracentrifugation onto a sucrose/D2O density cushion, and concentrated with buffer exchange by ultrafiltration/diafiltration. The volume was normalized to represent a 1000x (1uL equivalence = 1mL supernatant) concentration of the original supernatant volume.
   Figure 12a: Titration of anti-HLA/DR with excess exosomes. Figure 12b: Titration of dexosomes after ultracentrifugation into the cushion (UC-Cushion) and after diafiltration into formulation buffer (2nd UF) with excess anti-HLA/DR. Figure 12c: Titration of anti-MHC I (HC-10 hybridoma) with excess dexosomes. Figure 12d: Titration of dexosomes A and B with excess anti-MHC I.

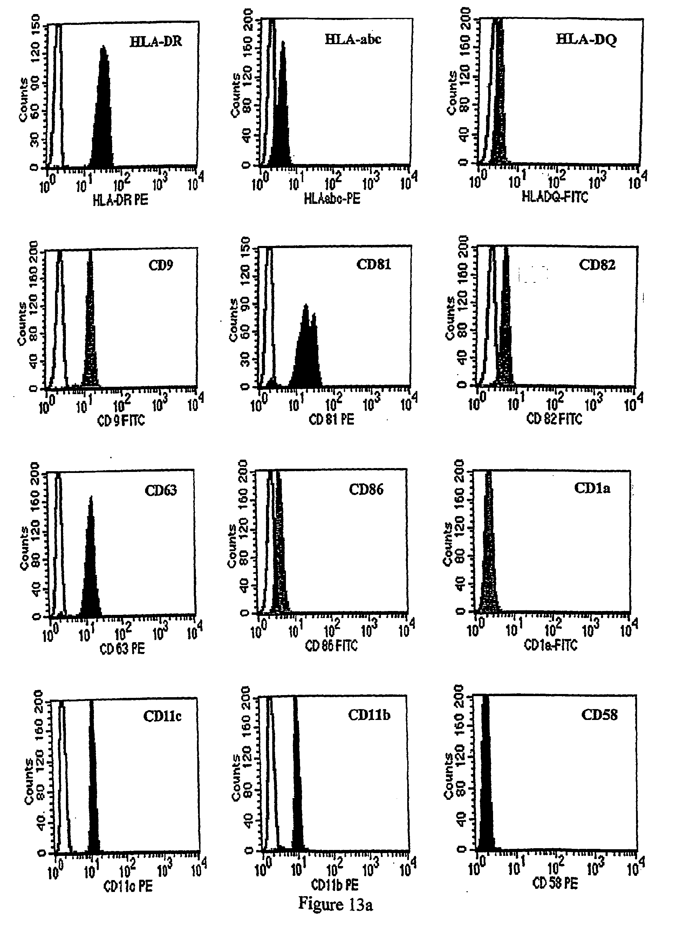

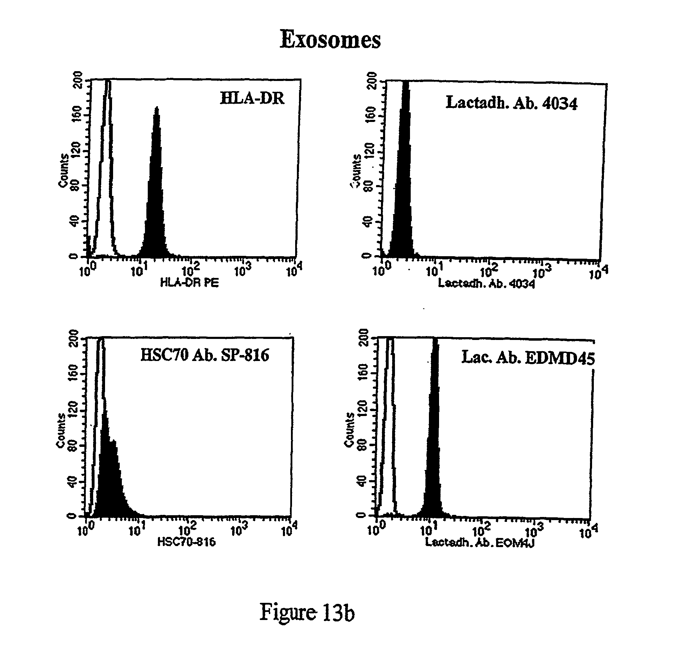

**Figure 13** Figure 13a: Flow cytometry analysis of Dexosomes for specific surface receptors. Shaded histograms illustrate specific intensity of binding of fluorescent labeled antibodies recognizing noted receptors and open histograms show irrelevant background binding. Figure 13b. As for Fig. 13a except that staining was performed using unlabelled primary antibodies followed by fluorescent labeled secondary antibodies since lactadherin antibody is not available as direct fluorescent conjugate.

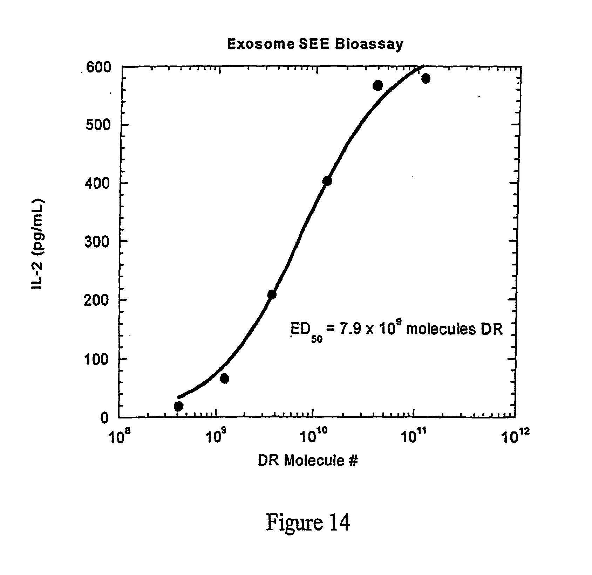

**Figure 14.** Superantigen assay using SEE-pulsed dexosomes. Dexosomes were purified and pulsed with SEE as described in the examples, then incubated with a combination of Jurkat T cells and Raji cells. Production of IL-2 was measured by ELISA and plotted as a function of number of HLA-DR molecules per well as determined by HLA-DR ELISA assay. The data was fitted using a sigmoidal curve fit and a calculated half-mazimal effective dose (ED50) was derived as a measure of the potency of the dexosome preparation.

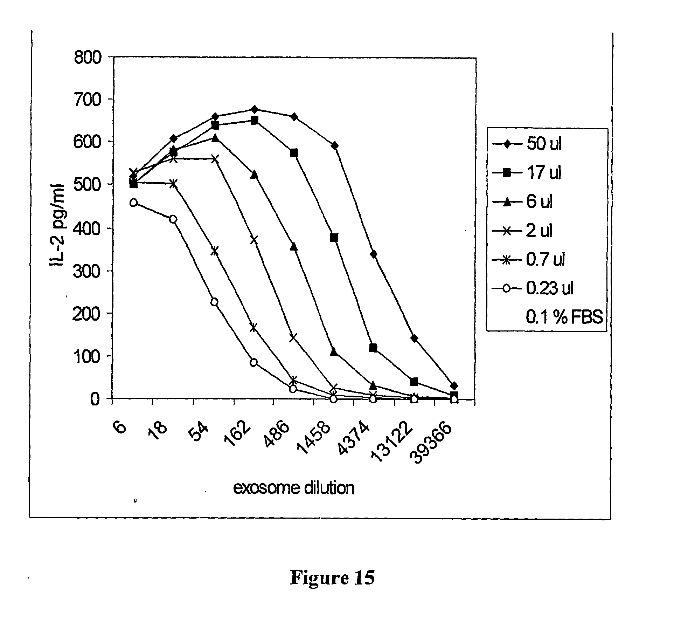

**Figure 15.** Diluted quantities of dexosomes (shown in the legend) were pulsed with SEE and cultured with Jurkat and Raji cells to test the potency of the Dex preparations. After recovery from the Optiprep cushion, each sample was subjected to a serial titration and tested in the cell assay. The data show that after correcting for the dilution of each dexosome preparation, the same level of activity is registered if one views the region of the curves registering approximately half-maximal activity (approximately 350 pg/ml IL-2) for the range of 0.7 ul to 50 ul of exosomes representing a range of nearly 100-fold over which the assay is linear. This result is unexpected for such an assay and makes the assay very useful to measure a wide range of concentrations of unknown Dexosome preparations.

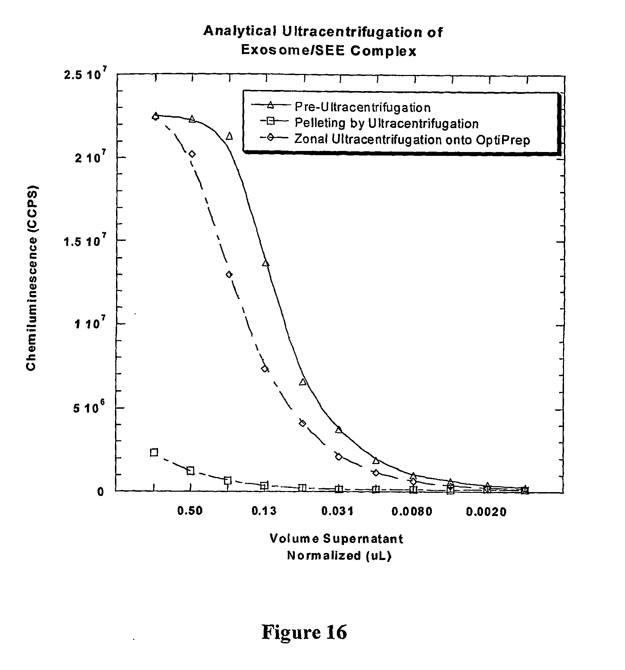

**Figure 16.** HLA/DR ELISA comparison of exosome/SEE complex purified by pelleting and zonal ultracentrifugation.

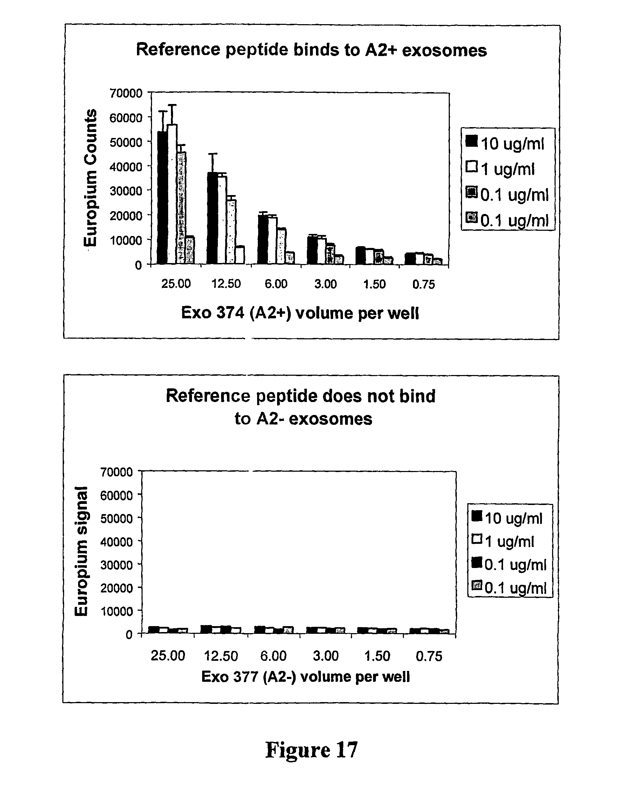

**Figure 17.** Peptide binding to HLA-A2+ (upper) and HLA-A2- (lower) dexosomes. Biotinylated reference peptide was used at the concentration indicated by the legend to load dexosomes. The amount of peptide bound is indicated on the y-axis in terms of Eu fluorescence (counts per second) as a function of the amount of dexosome lysate assayed (indicated in terms of µl volume on the x-axis).

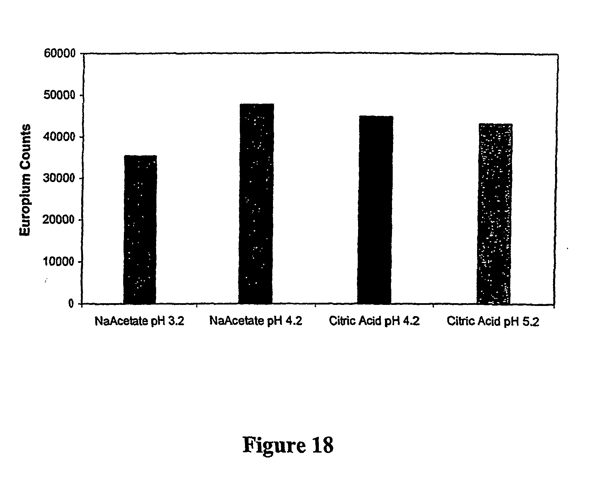

**Figure 18.** Peptide loading with different buffer and pH. Biotinylated reference peptide was loaded using acetate or citric buffer at pH 3.2 -5.2. The amount of peptide loaded is indicated on the y-axis in terms of Eu fluorescence counts.

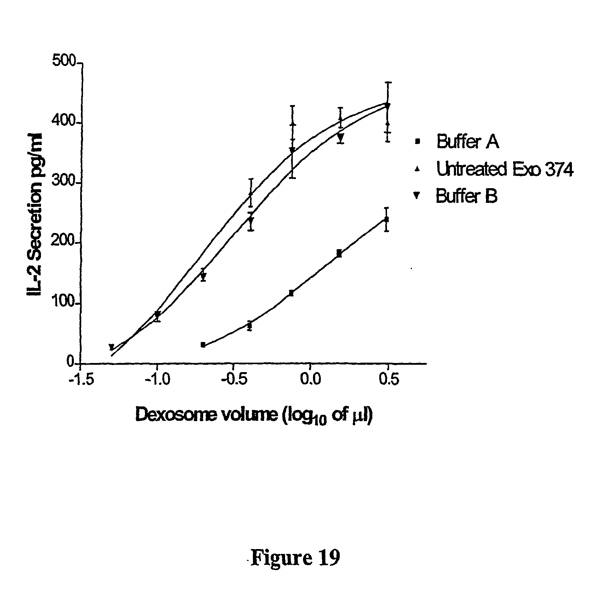

**Figure 19.** Dexosomes mild acid treated with pH 4.2 Na Acetate buffer retains better functional activity than pH 4.2 citric acid treated dex.

Dex were treated with either citric acid (Buffer A) or Na Acetate (Buffer B), pulsed with 'SEE' superantigen, and tested in a functional assay to evaluate effects on dexosome bioactivity as measured by their ability to elicit IL-2 secretion from effector cells. IL-2 secretion is indicated on the y axis as a function of dexosome volume.

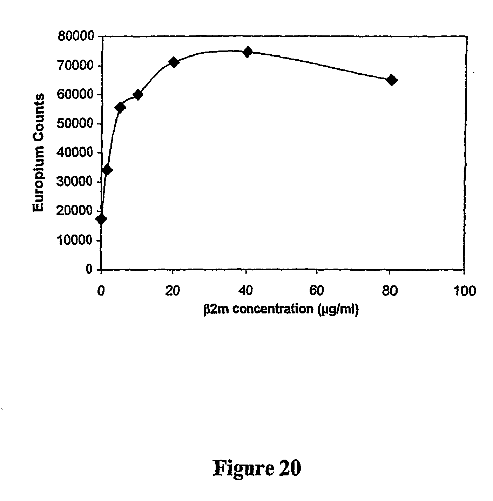

**Figure 20.** β2-microglobulin facilitates direct peptide loading. Biotinylated reference peptide was loaded with β2-microglobulin concentrations from 0-80 ug/ml. The amount of peptide bound is indicated on the y-axis in terms of Eu fluorescence (counts per second) as a function of the β2-microglobulin concentration.

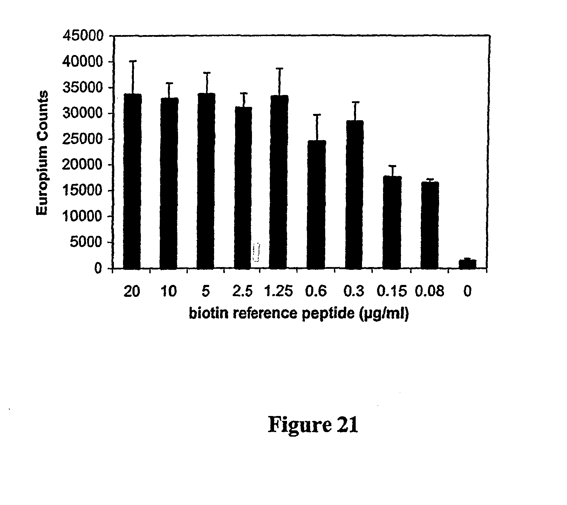

**Figure 21.** Saturation of the binding of reference peptide to dexosome. Biotinylated reference peptide was loaded at the concentrations of 0-20 µg/ml. The amount of peptide bound is indicated on the γ-axis in terms of Eu fluorescence (counts per second) as a function of the amount of reference peptide.

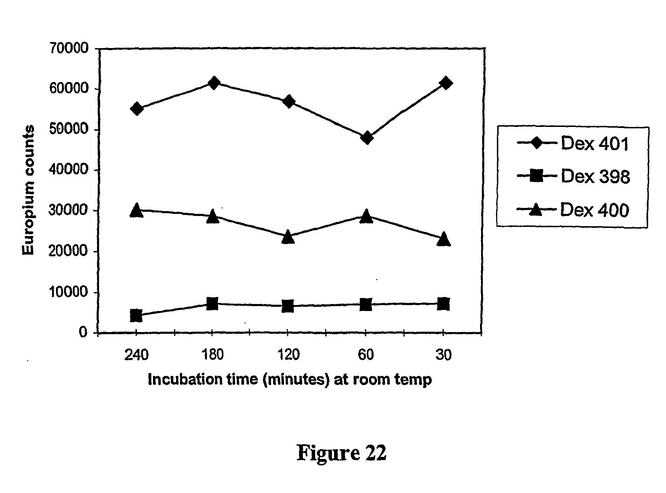

**Figure 22.** Kinetics of reference peptide binding to dexosome. Biotinylated reference peptide was loaded at room temperature from 0.5-4 hours. The amount of peptide bound is indicated on the y-axis in terms of Eu fluorescence (counts per second) as a function of the incubation time of reference peptide with dexosome

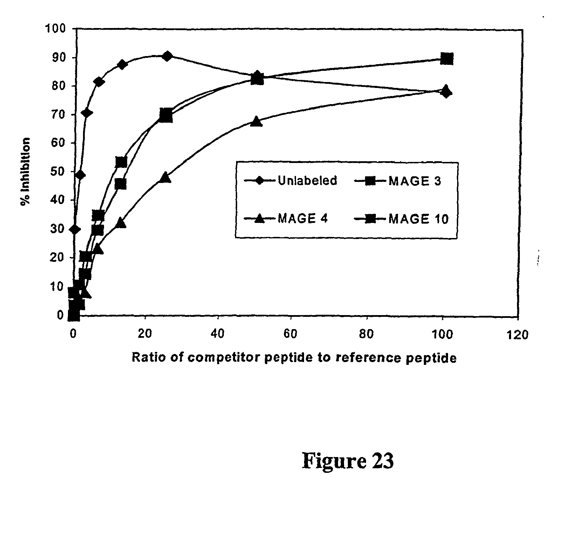

**Figure 23.** Competition of the binding of unlabeled reference peptide, MAGE3, 4, and 10 with biotinylated reference peptide on dexosome. 1-100 x of unlabeled reference peptide, MAGE-3, 4, and 10 were incubated with biotinylated reference peptide (5µg/ml) to compete for binding to dexosome. Percentage of inhibition is indicated on the y-axis as a function of the quantity of competitor peptide.

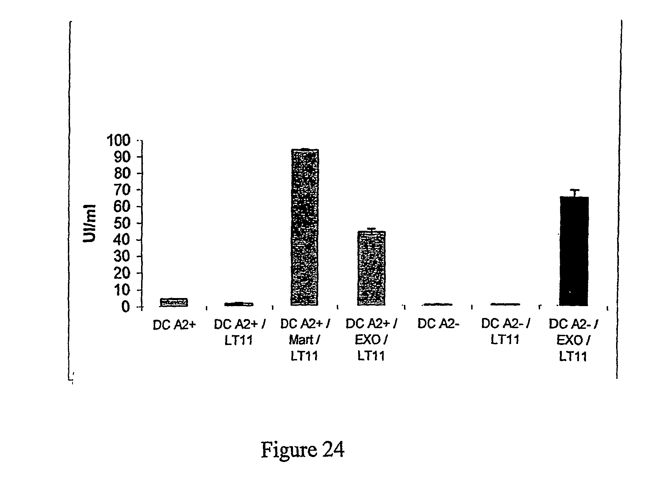

**Figure 24.** T Cell Clone Assay using Mart-1 loaded Dexosomes.

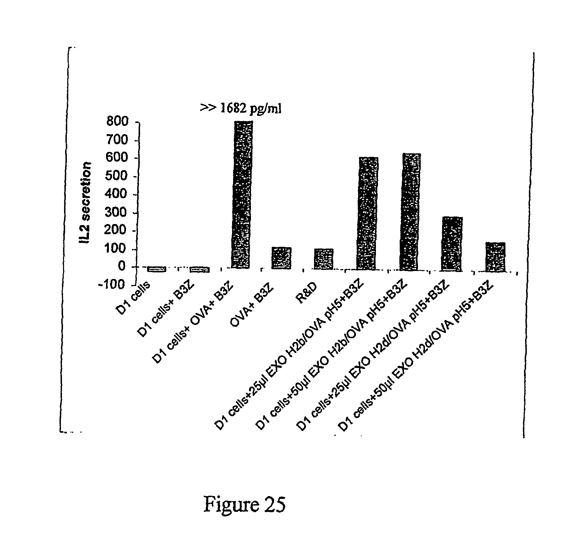

**Figure 25.** T cell Clone asssay using P1A-loaded Dexosomes. R&D is a 100 Unit IL-2 standard assay.

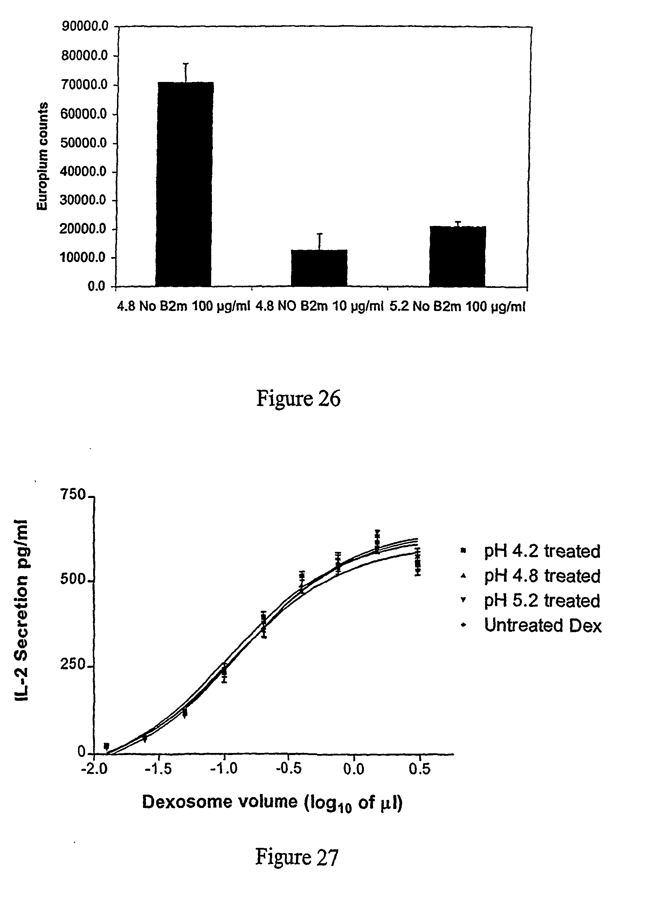

**Figure 26.** Comparison of the binding of biotin-labeled reference peptide to dexosomes in acetate buffer of pH 4.8 and 5.2 in the absence of beta 2 microglobulin. The amount of peptide loaded is indicated on the y-axis in terms of Eu fluorescence counts.
**Figure 27.** Dexosomes mild acid treated with pH 4.8 and 5.2 NaAcetate buffer retains the same functional activity as dexosomes untreated. Dex were treated with pH 4.8 or 5.2 NaAcetate(Buffer A), pulsed with 'SEE' superantigen, and tested in a functional assay to evaluate effects on dexosome bioactivity as measured by their ability to elicit IL-2 secretion from effector cells. IL-2 secretion is indicated on the y-axis as a function of dexosome volume.

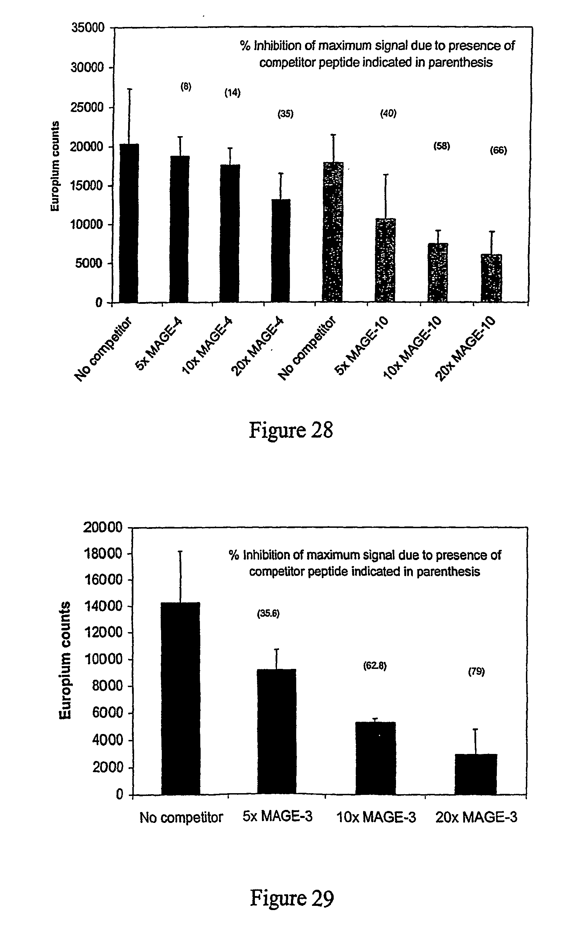

**Figure 28.** Competition of the binding of MAGE 4, and 10 with biotinylated reference peptide on dexosomes. 5-20 x of MAGE- 4, and 10 were incubated with 100 µg/ml of biotinylated reference peptide to compete for binding to dexosome at pH 4.8 in the absence of beta 2 microglobulin. Percentage of inhibition is indicated on the top of each column as a function of the quantity of competitor peptide.
**Figure 29.** Competition of the binding of MAGE 3 with biotinylated reference peptide on dexosomes. 5-20 x of MAGE-3 were incubated with 100 µg/ml of biotinylated reference peptide to compete for binding to dexosome at pH 4.8 in the absence of beta 2 microglobulin. Percentage of inhibition is indicated on the top of each column as a function of the quantity of competitor peptide.

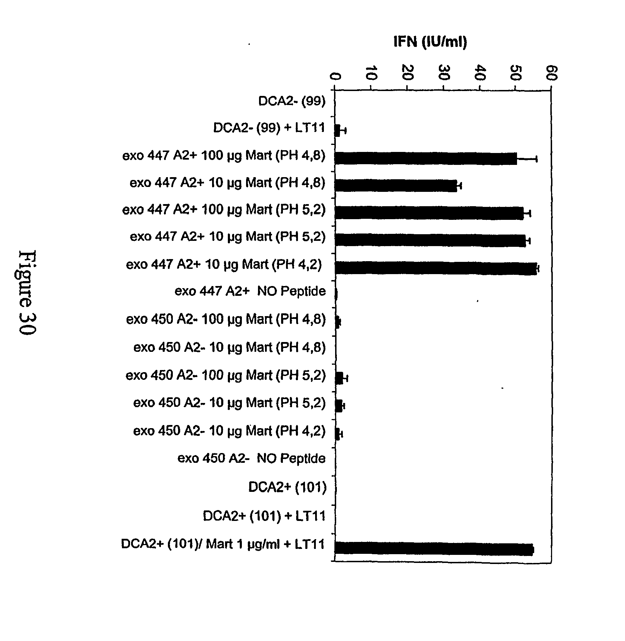

**Figure 30.** IFN-γ secretion by LT 11 stimulated by dexosome loaded with Mart-1 peptide in the absence of beta 2 microglobulin. LT 11 cells were stimulated by Mart-1 peptide loaded dexosomes in the presence of DC as accessory cells and IFN-γ secretion from LT 11 is indicated on the y-axis as a function of dexosomes. As control, Exo 447 was also loaded at pH 4.2 in the presence of beta 2 microglobulin.

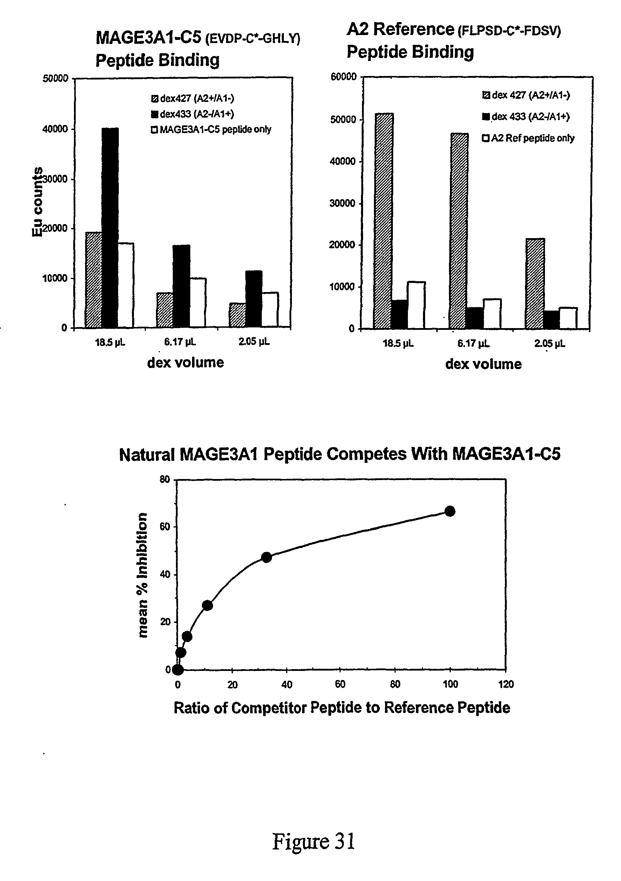

**Figure 31.** Binding (top panel) and inhibition (bottom panel) of HLA-A1 restricted peptide in the presenc of beta 2 microglobulin. Top panel: HLA-A1 restricted, biotin-labeled peptide MAGE-3C5 specifically bind to HLA-A1⁺ dexosomes. Bottom panel: the binding is inhibited by unlabeled MAGE-3A1 peptide.

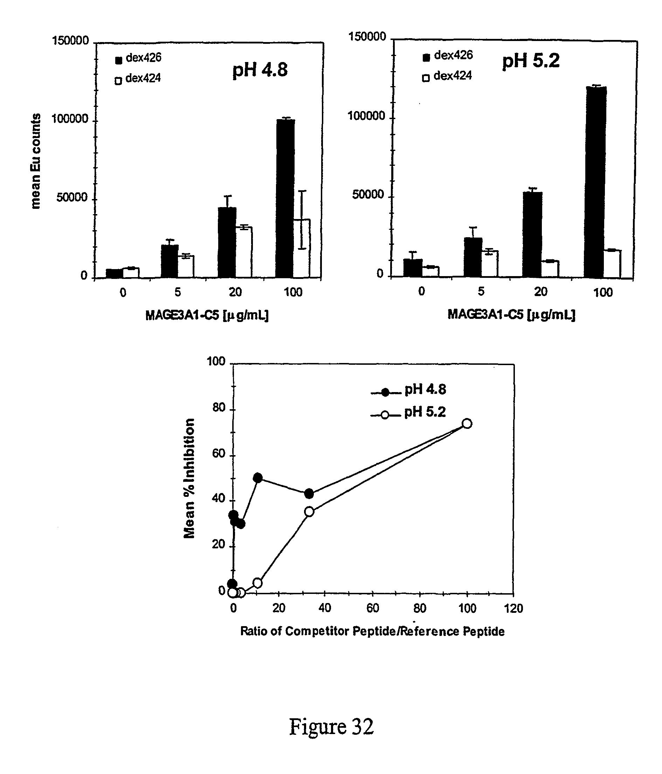

**Figure 32.** Competition between HLA-A1/B35 restricted, biotin-labeled MAGE-3C5 and unlabeled MAGE-3A1/B35 peptide on HLA-B35⁺ dexosomes (Exo 426) in the absence of beta 2 microglobulin.

### DETAILED DESCRIPTION OF THE INVENTION

As indicated above, the present invention provides novel methods to prepare and/or characterize membrane vesicles, which are suitable for use in pharmaceutical domains, such as immunotherapy of various pathological conditions. This description also discloses methods of removing haptoglobin aggregates from compositions such as media, biological products and the like, which can be used in various pharmaceutical or experimental areas.

Detailed description of the various steps that can be implemented to prepare membrane vesicles according to preferred specific embodiments of this invention will now be provided. It should be understood that the present invention is not limited to methods comprising all of these steps, but also include individual steps per se, as mentioned above.

A schematic representation of a complete process scheme for preparing membrane vesicles is depicted on Figure 1. This general process comprises several main phases, including (i) the production of the (biological) sample containing membrane vesicles, (ii) the enrichment phase, (iii) the density cushion separation phase, (iv) the formulation and conditioning phase and (v) the quality control or characterization phase. Furthermore, the invention also describes methods of removing haptoglobin from biological materials such as culture media.

### Haptoglobin aggregates removal

A particular and important aspect of the present disclosure resides in the provision of compositions that are essentially free of particulate bodies such as haptoglobin (and related polymers), more specifically of haptoblobin aggregates. In particular the present invention resides in the provision of cell culture media or biological products, such as proteins or polypeptides (or derivatives thereof) isolated from biological fluids (e.g., hSA compositions, gamma immunoglobulins, coagulation factors, serum, etc.) or buffer formulations, that are essentially free of aggregated haptoglobin.

Haptoglobin is a complex (alpha-beta)₂ tetrameric protein incorporating two types of alpha chains, alpha-1 (8.86 kDa) and alpha-2 (17.3 kDa), in various combination(s). It has been reported that haptoglobin can form large protein aggregates upon heat inactivation of serum products ("Biological Functions of Haptoglobin - New Pieces to an Old Puzzle". W. Dobryszycka, Eur.J.Clin.Chem.Biochem. 1997, 35(9), p.647-654 ; "Immunosuppressive Effect of Accute-Phase Reactant Proteins in vitro and its Relevance to Cancer", R. Samak et al., Cancer Immunol Immunother 1982, 13, p. 38-43). Furthermore, it is known that haptoglobin may exhibit immunogenic activity (Dobryszycka, supra, Oh et al., J. National Cancer Institute 1990, 82(11), p. 934-940). The present invention now recognizes the critical importance of haptoglobin aggregates that are present in many biological products and proposes new methods that allow the removal thereof.

More particularly, within the context of the present disclosure, haptoglobin aggregates designate any particulate body comprising a haptoglobin polypeptide or chain, more preferably cross-linked to any other polypeptide or protein through a S-S binding for instance, in particular any mixed aggregate of a haptoglobin polypeptide and albumin. Such haptoglobin aggregates may also comprise additional components such as hemopexin, transferring, Gc-Globulin and/or β₂-glycoprotein, as described by Jensen et al. (Vox Sang 67, 1994, 125).

While culture media are routinely used in both research and clinical applications, the issue of large soluble protein aggregates (or particulates) has not been addressed in the art, and their removal not suggested. The present disclosure now shows that the protein aggregates from AIM V co-purify with dexosomes upon pelleting via ultracentrifugation. Furthermore, this application shows that concentration of dexosomes generated in AIM V by ultrafiltration resulted in concentration of AIM V proteins. Furthermore, SDS-PAGE (Figure 3) of concentrated dexosomes depicted 2 major bands at 42kDa and 64kDa corresponding to haptoglobin β-chain and albumin.

This disclosure thus demonstrates that haptoglobin aggregates (including sub-units, cross-linked chains, aggregated forms, etc.) is found in conventional mammalian cell culture media. This disclosure further shows that haptoglobin aggregates are not removed by conventional exosome purification methods. The present disclosure now provides methods of removing particulate bodies, more preferably haptoglobin aggregates from biological materials such as culture media, biological compositions, buffering formulations, etc. The disclosure also discloses novel compositions of matter that are essentially deprived of haptoglobin aggregates and their uses. Removal (or reduction of the concentration) of large protein aggregates such as haptoglobin aggregates provides several significant advantages such as increased purity, increased safety, reduced non-specific immunosuppressive activity, etc. Furthermore, the disclosure discloses that aggregated haptoglobin-free culture media still allow an efficient cell culture and production of exosomes, while greatly enhancing the purification of the exosomes. Removal of protein aggregates from the media offers additional safeguards against the induction of undesirable immune responses to serum components such as haptoglobin (Dobryszycka, supra). In this regard, it is believed that haptoglobin aggregates can cause more undesirable immune response than non aggregated haptoglobin which is already known to be immunosupressive (Se-Kyung Oh et al J. Natl Cancer Inst. 1990, 82, 934-940 Interference with immune response at the level of generating effector cells by tumor-associated haptoglobin). Protein aggregates are known to be 10,000 (ten thousand) times more immunogenic than soluble forms because they are preferentially captured by antigen presenting cells (M. Kovacsovics-Bankowski et al Proc Natl Acad Sci USA 1993, 90, 4942-4946 Efficient major histocompatibility complex class I presentation of exogenous antigen upon phagocytosis by macrophage). So, even present at very low dose, haptoglobin aggregates could be deleterious.

Furthermore, the methods of this disclosure also allow to remove other particulate bodies such as exosomes which may be present in serum-containing culture media. Removal of pre-existing exosomes further increases the purification of the products and avoids contamination by other immune-stimulating agents.

### Method of removal

Various methods can be used for removing particulate bodies (e.g., protein aggregates, more particularly haptoglobin aggregates) such as (ultra)filtration, microfiltration, size exclusion chromatography (SEC), affinity chromatography, ion exchange chromatography, and ultracentrifugation. It may also be possible to remove the protein aggregates in the human serum component (i.e. Fraction V) by these methods prior to formulating with the medium.

In a preferred embodiment, the medium or composition is treated by ultrafiltration to remove protein aggregates or other particulate bodies, more specifically aggregated haptoglobin.

In a particular embodiment, ultrafiltration is performed using a 500kDa hollow fiber membrane. This is the preferred molecular weight cutoff (MWCO) for this application since proteins such as transferrin (approximately 75-80 kDa) are required to pass through the membrane. Measurements of transferrin concentration by ELISA indicate that it quantitatively passes through this pore size. This membrane size retained the protein aggregates in the retentate while allowing the un-aggregated proteins to pass through with the permeate. The permeate is collected, sterile filtered through 0.22µm filter into bottles, and stored at 4°C until use.

While a 500kDa MWCO hollow fiber membrane is preferred for ultrafiltration of culture media, other sizes of membrane may also be used such as 750kDa, 300kDa, 100kDa, etc. Preferably, the medium is ultrafiltered with a membrane having a diameter comprised between 100kDa and 1000 kDa, more preferably between 200 kDa and 750 kDa. In this respect, Applicants have now determined that 90% of haptoglobin aggregates present in culture media or other biological products such as heated serum albumin have a diameter comprised between about 40 nm and about 200 nm, as measured by dynamic light scattering. Accordingly, any membrane (or other separation device such as filters, hollow fibers, etc.) having a pore diameter below about 40 nm would be suitable and preferred for performing the present invention. As an illustration, a MWCO of 500 kDa represents a pore diameter comprised between about 20-25 nm.

Also, while the hollow fiber membrane format is the preferred embodiment for ultrafiltration, other types of ultrafiltration devices in a cassette format (i.e. plate or frame cassette) may be used such as the Millipore Pelicon and related products, as well as cassettes from Sartorius Inc or Filtronics Inc. The membrane material is typically composed of polyether sulfone (PES), however other materials such as polypropylene may also be used.

In addition, a large range of operating parameters may be used in ultrafiltration of the media. Typical operating parameters are such that the inlet and outlet pressures are between 5-15 psi for optimal processing. A much lower or higher pressure may be used during processing.

In another specific embodiment, the medium is treated by microfiltration (0.05µm, 0.1µm, 0.2µm, etc) to remove particulate bodies (e.g., protein aggregates). The preferred lumen diameter of the fiber is 0.5mm, however other diameters such as 0.25mm, 0.75mm, and 1mm may also be used.

These various treatments allow the production of media or biological products with reduced particulate bodies content, which have now been shown to significantly enhance the purification of the exosomes as well as the quality of the resulting preparation.

### Aggregated Haptoglobin-free Media

In a particular aspect, the present disclosure thus resides in the use of pre-treated culture media, having a reduced particulate bodies content, as well as compositions of matter comprising such pre-treated media. Indeed, it has now been shown that pre-treatment of the media to reduce the content of particulate proteins (or protein aggregates) reduces significantly the contaminants present in the process, increases the efficacy of the method, allows the production of exosome preparations with higher purity and safety, and does not affect culture efficacy or cell viability.

Accordingly, in a particular embodiment of the present disclosure membrane vesicles are prepared from biological samples produced by cells cultured in a culture medium with reduced particulate bodies (or protein aggregates) content.

Furthermore, another object of this disclosure, resides in a method of producing dendritic cells, comprising culturing dendritic cell precursors in a medium comprising growth factors and/or cytokines to effect or stimulate differentiation of said precursors into dendritic cells, in particular into immature dendritic cells, wherein the medium has a reduced particulate bodies content, more preferably is essentially free of haptoglobin aggregates.

The culture or production medium may be any medium suitable for culturing mammalian cells, in particular human cells. Examples of such media include AIM V, RPMI, DMEM, and the like, more generally any mammalian cell culture medium comprising proteins (or serum or substitute thereof). Preferred media include serum-free media, which are suitable for clinical uses.

The term "essentially free", "deprived of' or "reduced content in" haptoglobin aggregates indicates that the medium or product contains preferably less than 1ppm of haptoglobin aggregates, more preferably less than 0.5ppm by weight of aggregated haptoglobin. More particularly, the Applicants have now determined, by SDS PAGE and ELISA, that above 99% of haptoglobin aggregates could be removed from biological products such as AIM V media. More specifically, the culture medium is essentially devoid of particulate bodies (including protein aggregates, precipitates, and the like) having a diameter above 100 nm. In a further preferred embodiment, the medium is essentially devoid of particulate bodies (including protein aggregates, precipitates, and the like) that do not pass through a 500 kDa membrane. A more preferred culture medium of this invention is a culture medium that contains less than about 20 ng/ml, more preferably less than about 10 ng/ml of aggregated haptoglobin, as determined by ELISA (using for instance monoclonal antibody H6395 of Sigma). Furthermore, it should be noted that the presence of haptoglobin aggregates is generally not noticeable until the media is concentrated (by ultracentrifugation or ultrafiltration). A preferred media of this invention thus does not contain haptoglobin aggregates, as measured by SDS PAGE analysis and ELISA.

### Aggregated Haptoglobin-free Biological products

The disclosure is also suitable for the production of compositions of biological products (e.g., blood products) that are essentially free of aggregated haptoglobin, as well as to the treatment of various buffer solutions prior to formulating products for pharmaceutical uses.

In this respect, the present disclosure relates to a composition comprising a biological polypeptide or a derivative thereof, that is essentially deprived of haptoglobin aggregates. More particularly, this invention resides in a composition comprising a heat inactivated biological polypeptide that is essentially deprived of haptoglobin aggregates. The biological polypeptide may be any polypeptide, protein or peptide isolated (or extracted) from a mammalian biological fluid, in particular from blood, serum or plasma. Preferred examples of biological polypeptides include serum-albumin, gamma immunoglobulin, coagulation factors (e.g. factor VIII, factor IX), more preferably of human origin. The composition of the present invention are even more preferably characterized by containing less than about 0.01%, particularly less than about 0.001% haptoglobin aggregates.

In a specific example, this disclosure relates to a composition of heat inactivated serum-albumin, more preferably human serum-albumin, essentially free of aggregated haptoglobin. Purified hSA is widely used in the pharmaceutical industry (plasma complement, protein stabilizing agent, etc.). The presence of haptoglobin in hSA solution was demonstrated to be responsible for the formation of aggregates during heat treatment. Furthermore, aggregated proteins seem to be at least ten thousand times more immunogenic than un-aggregated forms thereof, so that their presence might be deleterious to the activity and safety of the preparation. The present disclosure now allows to remove any such haptoglobin aggregate from hSA preparations, more particularly through ultrafiltration as described above, thereby providing novel hSA compositions with high purity and quality for pharmaceutical uses. More particularly, the invention now provides hSA preparations that contain less than about 0.01% by weight (wt) of aggregated haptoglobin, even more preferably less than about 0.001% wt. The specific examples described in this application demonstrate that albumin preparations containing about 0.00025% wt aggregated haptoglobin, or less, may be obtained with the present invention. More preferably, the albumin preparations (or compositions) of this invention are heated hSA preparations (or compositions), especially for pharmaceutical uses.

The disclosure also resides in a method of treating a biological product, more preferably a heat inactivated biological product, in order to reduce the amount of haptoglobin aggregates contained therein, the method comprising subjecting the product to filtration, more preferably ultrafiltration.

A particular object of this disclosure also resides in a method of preparing a biological product comprising (i) a heat inactivation of the biological product and (ii) a filtration of the heat inactivated biological product. More preferably, the method further comprises the step of (iii) concentrating the filtered, heat inactivated biological product and/or (iv) the conditioning thereof. The method can be used for various biological products including any protein or polypeptide (or derivatives thereof) isolated (or extracted) from mammalian biological fluids such as human blood or plasma or serum. As will be further documented in this application, this method allows, for the first time, the production of heat inactivated biological products having a reduced content in haptoglobin aggregates, more preferably essentially free of haptoglobin aggregates and thus with increased safety. The method is particularly suited for the preparation of pharmaceutical proteins extracted from blood or plasma such as serum-albumin, more preferably a human serum-albumin, gamma immunoglobulin, coagulation factors, etc. The filtration step comprises preferably an ultrafiltration, even more preferably with a MWCO comprised between about 100 kDa and about 1000 kDa, typically between 200 kDa and 750 kDa. The filtration may be conducted in any device and condition as disclosed above. Furthermore, alternative methods as described above may also be used.

In this regard, the disclosure also relates to methods of treating albumin preparations comprising subjecting an albumin preparation to filtration, preferably ultrafiltration. A more preferred method resides in the treatment of a heated hSA composition (or preparation) by ultrafiltration on a porous device having a mean pore diameter comprised between 200 kDa and 750 kDa. Because of the large amounts of hSA used in the pharmaceutical area, Applicants' method and compositions of higher quality represent significant advantage in terms of security.

### Production of the Sample

As indicated above, the current disclosure relates to the production of membrane vesicles and is suitable to prepare membrane vesicles from various origins, including membrane vesicles produced by antigen-presenting cells (such as macrophages, dendritic cells, B lymphocytes), tumor cells or any other cell or cell line producing membrane vesicles. It is particularly suited for preparing membrane vesicles produced by dendritic cells, preferably immature dendritic cells (i.e., dexosomes). Furthermore, the membrane vesicles or corresponding producing cells can be sensitized to one or several antigens, prior to, during or after preparation.

### Monocyte Cell Culture

Various methods of producing biological samples containing dexosomes or other membrane vesicles have been disclosed in WO99/03499, incorporated therein by reference.

A preferred methodology within the scope of this disclosure is based on the production of dendritic cells ("DC") from monocyte precursors or bone marrow, more preferably immature DC. Indeed, the inventors have shown that immature DC have the capacity to produce exosomes, while mature DC essentially fail to do so. More specifically, it is preferred to use compositions of immature dendritic cells obtained by treating monocyte precursors (contained in blood or marrow) in the presence of a combination of cytokines, more preferably in the absence of DC maturation factor or condition, and/or for a period of time that does not allow DC maturation.

Compositions of immature dendritic cells preferably comprise essentially (i.e. at least 60%, preferably 70%) immature dendritic cells.

Therefore, the dendritic cell preparation step advantageously comprises the preparation of a composition of immature dendritic cells, particularly of human origin, especially from monocyte precursors, more specifically by treatment with a combination of cytokines such as GM-CSF+IL-4 or GM-CSF+IL-13, in the absence of maturation factors and/or in serum-free media to avoid maturation.

In addition, it is also possible to use immortalised dendritic cell populations. These may consist of immortalized dendritic cell lines (e.g. D1 line or any other line produced by introducing the myc oncogene in the dendritic cells, for example). They may also consist of dendritic cells prepared and immortalized in vitro. The interest of immortalized dendritic cells lies in the constitution of banks of cells sensitised to given antigen groups, which may be used industrially to prepare dexosomes compatible for administration to entire families of patients.

To produce the membrane vesicles (dexosomes), the immature dendritic cell population may be simply cultured under conventional conditions known to those skilled in the field. However, it is preferred to culture these cells under conditions stimulating the production of dexosomes, particularly in the presence of factors capable of stimulating dexosome production, particularly a cytokine such as gamma interferon, interleukin 10 or interleukin 12 (e.g. see application WO99/03499). In a preferred embodiment of the process, the immature dendritic cell population is cultured under conditions stimulating membrane vesicle production. Preliminary experiments indicate addition of interferon gamma enhances the efficacy of dexosomes in vivo in preclinical mouse tumor models. On day 7, the media is collected for subsequent dexosome isolation.

In a specific embodiment, the patients' peripheral blood samples are cultured in clinical grade AIM V, a serum-free cell culture medium (Life Technologies, Inc). Prior to use, the culture medium undergoes ultrafiltration to remove aggregated proteins (e.g., haptoglobin), whose removal was shown by applicants not to affect cell growth, but aids considerably in the subsequent isolation of pure dexosomes.

It is understood that membrane-vesicles can be prepared by any other means. In particular, membrane vesicles may be produced artificially, or with immortalized cell lines or obtained from previously established collections or banks.

### Sensitization of the cells or vesicles : Antigen Loading

The membrane vesicle-producing cells (e.g., dendritic cells) can be sensitized to an antigen prior to (or during) membrane vesicle production. Alternatively, the membrane vesicles themselves may be sensitised. This embodiment allows the production of vesicles with a given immunogenicity. The sensitisation may be performed using different well-known techniques, comprising for example placing the cells in contact with antigenic peptides, antigens, protein complexes, cells or membranes of cells expressing antigens, apoptotic bodies, membrane vesicles, liposomes, tumoral RNA or any nucleic acid coding for one or more antigens, antigenic determinants or epitopes (possibly carried by a viral or non-viral vector), etc. (e.g. see application WO99/03499). Alternatively, the sensitisation may be performed by direct peptide loading of the vesicles, i.e., by placing the vesicles in contact with antigenic peptides. Indeed, the present application shows that direct peptide loading of vesicles is possible and provides an improved immunogeniciy to the vesicles. In a preferred method, the sensitisation is performed by incubation of the producing cells with peptides, antigens, RNA or nucleic acids or by direct peptide loading of the vesicles. It is understood that this application is not limited to sensitisation or production techniques.

Many antigens can be used to sensitize dendritic cells (or membrane vesicles), by exposure of the cells (or vesicles) to said antigens, corresponding proteins, peptides, nucleic acids and the like. Preferred antigens are tumor antigens, viral antigens, bacterial antigens and the like. Typical tumor antigens include melanoma antigens (MAGE, MART, BAGE, etc), prostate-specific antigens (e.g. PSMA), CEA, ras, p53, Rb, liver tumor antigens, etc.

Protein antigens are processed inside dendritic cells ("DC") to specific peptides, which are then captured by the MHC molecules for presentation at the cell surface. For a given protein, human DC of different HLA haplotypes present different peptides (epitopes). Relevant peptides can be loaded to the MHC class I of DC by incubating DC and peptides under the proper conditions. It is also possible to load antigens directly on the isolated dexosomes in vitro. In a specific example, MAGE-A3 and -A4 peptides comprising known epitopes for HLA-A2 are synthesized for use in loading the patients' dexosomes. Inclusion criteria for patient in this experiment include expression of the HLA-A2 haplotype. The HLA A2⁺ haplotype is relatively frequent, being present in approximately 50% of the human population.

Furthermore, control antigens such as TT (tetanus toxoid) and CMV peptides can also be added, in particular the TT P2 peptide. The purpose of the control antigens is to serve as an internal control to test the function of dexosome in antigen presentation with known antigens. Therefore, as a positive control, dexosomes are also loaded for instance with the CMV peptide (class I) and TT P2 peptide (class II). A positive response with these controls indicates that the dexosomes are active. CMV has the advantage of providing a test for both the initial and recall antigen response because approximately 50% of the general population are immune to CMV (recall) and the remainder is naïve (initial).

Other antigens are lipid antigens such as any lipid, glycolipid or lipoprotein presented to an immune system by CD1 molecules. The lipid may be any microbial lipid, microbial glycolipid, lipid or glycolipid tumor antigens, etc. Specific examples of lipids include mycobacterial lipids such as mycolic acids, glucose monomycolate, hexose-1-phosphoisoprenoids and derivatives of lipoarabinomannans (LAM) such as phosphatidylinositolmannosides. Other examples include self ceramides such as gangliosides, glycolipid tumor antigens, etc.

Example 3 below describes preliminary results obtained in a model system for the loading and assay of CMV peptide (antigen) on dendritic cells and dexosomes. The results demonstrate the ability of loaded vesicles to stimulate antigen-specific CTLs (Figure 2).

It should be understood that any other sensitization method and antigen(s) may be used to confer desired immunogenic properties to the membrane vesicles.

In this regard, the disclosure also describes method of direct loading of membrane vesicles, e.g., dexosomes. The method can be performed using class I or Class II peptides or lipids, and provides significant advantages over prior techniques. In particular, as illustrated int the examples, the direct loading is more efficient than prior indirect loading, in that a higher occupancy rate of surface HLA receptors can be obtained using lower amounts of peptide. This clearly increases the immunogenic potential of the membrane vesicles. In addition, the structure of the loaded peptide can be controlled more precisely, since the loaded peptides are not processed by whole cells and thus remain intact and un-modified upon loading. The disclosure now proposes and shows for the first time that direct loading can be carried out using purified membrane vesicles such as dexosomes. In this regard, the invention shows that membrane vesicles stand low pH conditions without loosing their activity and functionality. The disclosure also describes particular conditions allowing improved direct loading. The disclosure particularly show that conventional buffer media used for indirect loading (i.e., loading of whole cells) may advantageously be replaced with different buffer media to increase the direct loading efficiency and obtain higher occupancy rates, indicating that vesicles do not behave as whole cells. The disclosure also shows for the first time that, in contrast with whole cells, membrane vesicles may resist prolonged acid treatments or media, thereby allowing direct loading by replacement, even in the absence of added β2-m.

In a first variant, the method of direct peptide loading comprises the step of (i) subjecting the isolated or purified membrane vesicles to a selected acid medium and (ii) contacting the membrane vesicles of (i), preferably during or after neutralization, with the selected peptide in the presence of beta2-microglobulin, so that said peptide is loaded onto said membrane vesicles. In a further preferred embodiment, the selected peptide is a tumor antigen peptide presented by class I molecules, i.e., a class-I restricted tumor peptide.

In this first variant, the membrane vesicles (e.g., dexosomes) are first subjected to a selected acid medium. This acid elution removes at least a portion of endogenous peptides and beta 2-microglobulin (β2-m) associated with HLA class I or class II molecules at the surface of the vesicles, without altering the biological and immunological properties of exosomes. This treatment is advantageous since it avoids or reduces secondary immune responses directed against endogenous peptides. As determined by Applicants, the selected acid medium or treatment preferably comprises subjecting the vesicles to a medium with a pH comprised between 2 and 6, more preferably between 3 and 5,5. The present application indeed shows that exosomes can be treated at such acid pH and still retain their biological properties. In particular, the application surprisingly shows that exosomes can be subjected to mild acid treatment, neutralized and loaded with desired peptides and still exhibit the ability to stimulate effective immune response against the desired peptide. The medium may further comprise any suitable buffer solution, such as citrate, acetate, etc. In this regard, the inventors have shown that a much preferred medium comprises sodium acetate, which provides increased loading performance, as compared to conventional citrate buffer. The selected media may be derived from any conventional culture or growth medium, and prepared by adding thereto buffering conditions to reach a pH as described. The media may also be artificial or defined, using various buffer nutrients according to methods known in the art. Essentially, selected acid media according to the present invention designate any culture, growth, conditioning or other liquid solution or buffer allowing to maintain membrane vesicles, the solution having a pH as described above and, preferably comprising acetate, e.g., sodium acetate.

The mild acid treatment is performed at low temperature, typically below 8°C, preferably at about 4°C, for a period of time essentially of 15 minutes or less, preferably 10 minutes or less, even more preferably 5 minutes or less. It should be understood that the conditions of the treatment (medium, pH, temperature, duration, etc.) can be adjusted by the skilled artisan using the teaching of the present application and examples.
Neutralization can be performed according to conventional methods, by increasing the pH of the solution. Neutralization preferably results in a medium with a pH above 5.5, typically comprised between about 6 and about 9. Neutralization is typically performed by adding to the medium a neutralization medium at pH of about 10 or 11, for instance a Tris buffered solution.
Contacting of the peptide and exosomes shall be made under conditions sufficient to allow the peptide to bind MHC molecule on the surface of the exosome. Typically, the exogenous (Class I) peptide and β2-m are added, during or after neutralization, preferably in excess to favor the reconstitution of HLA class I after forming complexes with peptide and β2-m so that the peptide is loaded. It is not necessary to eliminate dissociated endogenous peptides before neutralization, or to remove unbound peptides after contacting. Typically, from 0.005 to 50 µg/ml of exogenous peptide (preferably from 0.01 to 10 µg/ml) and from 1 to 80 µg/ml of β2-m may be used. The peptide and β2-m may be produced by various techniques, such as recombinant production, synthesis, etc. It should be understood that β2-m is preferably of human origin (human β2-m is commercially available (Sigma) and can be produced by conventional techniques). Contacting may be performed for up to about 2 hours, for instance at room temperature. The examples show that saturation is obtained as early as 30 minutes post contacting. Using TRF with a biotinylated reference peptide, we can determine the amount of peptide directly loaded onto dexosomes and demonstrate that there is competition of binding between the labeled reference peptide and unlabeled target peptides, indicating that direct loading of target peptides to dexosomes is successful. Dexosomes loaded in this manner with Mart-1 peptide at concentrations as low as 0.1 µg/ml consistently and reproducibly stimulate a Mart-1 specific CTL clone LT 11 to produce IL-2 (Example 12).

In an other variant, the method does not require the use of beta2-microglobulin. Indeed, the inventors have shown that it is possible to define suitable conditions to remove endogenous peptides without drastically removing or destabilizing endogenous β2-m, so that direct loading is possible even without using exogenous β2-m. In this preferred variant, the method comprises the steps of (i) contacting an isolated or purified membrane vesicle with a class I-restricted peptide or lipid in the absence of beta2-microglobulin, (ii) subjecting the mixture of (i) to a selected acid treatment under conditions allowing the peptide or lipid to complex with an HLA class I molecule at the surface of said membrane vesicle, and (iii) collecting the loaded membrane vesicle. More preferably, step (ii) comprises subjecting the mixture to mild acid treatment at a pH comprised between about 4 and 5.5. for less than 2 hours, even more preferably between about 4.2 and 5.2, for less than 1 hour. This embodiment is advantageous since less treatments are needed and there is no need for exogenous β2-m, which reduces the costs, manipulation and potential problems associated with human-derived products. In this beta2-microglobulin-free embodiment, the isolated or purified membrane vesicle is typically contacted with an excess of exogenous peptide, e.g., with 5 to 500 µg/ml of class I-restricted peptide in the absence of beta2-microglobulin. Upon contacting under appropriate conditions as defined above, the loading occurs through a replacement of endogenous compounds associated with antigen-presenting molecules (e.g., MHC class I or II for peptides and CD1 for lipids) by the peptide or lipid of interest (exchange process). This exchange is rendered possible or sufficient by the presence of selected acid conditions defined by the inventors. The exchange is also rendered possible by the demonstration, by Applicants, that membrane vesicles such as exosomes resist prolonged aid exposure, in contrast with whole cells, and can thus be subjected to a defined acid treatment sufficient to facilitate the replacement step without adding exogenous β2-m. Once the exchange or replacement is performed, usually after 15 to 30 minutes (or more if necessary, or less depending on the conditions), the loaded vesicles may be stabilized by increasing the pH of the solution, thereby blocking the exchange. Neutralization may be performed as described above.

As indicated above, the method can be performed using various immunogenic compounds, such as peptides or lipids. The peptides are preferably peptides presented by class I or Class II molecules, i.e., peptides that result from processing of antigens by antigen presenting cells such as dendritic cells and macrophages and B lymphocytes. A preferred class of peptides is represented by class-I restricted peptides, even more preferably class-I restricted tumor peptides, i.e., peptides derived from tumor antigens, which are presented by dendritic cells or macrophages to the immune system. It should be understood that the method can be performed using isolated peptides or mixtures thereof, such as peptide eluates of tumor cells or other peptide combinations. Specific examples of lipids include bacterial lipids and glycolipid tumor antigens, as described above.

Example 12 below describes the results of direct loading of dexosomes. The results demonstrate the ability of loaded vesicles to stimulate antigen-specific CTLs.

### Enrichment of the Sample

As indicated above, the sample (e.g. supernatant, lysate, biological fluid, etc.) may be subjected to an enrichment step, which may comprise one or more centrifugation, clarification, ultrafiltration, nanofiltration, affinity chromatography and/or diafiltration steps. In a specific embodiment, the enrichment step comprises (i) the elimination of cells and/or cell debris (clarification), possibly followed by (ii) a concentration and/or diafiltration step. A preferred enrichment step according to this invention comprises (i) the elimination of cells and/or cell debris (clarification), (ii) a concentration, and (iii) diafiltration.

### Clarification of the sample

The cells and/or cell debris may be eliminated by centrifugation of the sample, for example, at a low speed, preferably below 1000 g, between 100 and 700 g, for example. Preferred centrifugation conditions during this step are approximately 300 g or 600 g for a period between 1 and 15 minutes, for example.

Preferably, the cells and/or cell debris are eliminated by filtration of the sample, possibly combined with the centrifugation described above. The filtration may particularly be performed with successive filtrations using filters with a decreasing porosity. For this purpose, filters with a porosity above 0.2 µm, e.g. between 0.2 and 10 µm, are preferentially used. It is particularly possible to use a succession of filters with a porosity of 10 µm, 1 µm, 0.5 µm followed by 0.22 µm.

Cell culture media is filtered by microfiltration through a disposable 0.8µm capsule filter composed of cellulose acetate to remove cells and debris. The microfiltration capsule is single use. A scheme of the process is shown in Figure 5. A minimum of 10 experiments indicate that the capacity of the microfilter, e.g. 500 cm² surface area, is sufficient for filtering dendritic cell culture supernatants of 2.5 liters to greater than 4 liters. If necessary a larger surface area (e.g. 1000cm²) may be used depending upon the amount of debris in suspension.

Other pore sizes 2µm, 1µm, 0.65µm, 0.45µm, etc may also be used to clarify cells and cell debris from the dexosomes. The microfilter may contain a pre-filter, i.e. the 0.8µm Sartoclean CA (Sartorius Inc.) contains a 3.0µm cellulose acetate pre-filter. Furthermore, the filter may be composed of other materials besides cellulose acetate such as polypropylene or polyether sulfone (PES).

Other methods that may be used for the removal of cells and debris from dexosomes are low speed centrifugation, continuous flow microfiltration through hollow fibers, and chromatography.

### Concentration

A concentration step may be performed, in order to reduce the volumes of sample to be treated during the density cushion stage. In this way, the concentration may be obtained by centrifugation of the sample at high speeds, e.g. between 10,000 and 100,000 g, to cause the sedimentation of the membrane vesicles. This may consist of a series of differential centrifugations, with the last centrifugation performed at approximately 70,000 g. The membrane vesicles in the pellet obtained may be taken up with a smaller volume and in a suitable buffer for the subsequent steps of the process.

The concentration step is preferentially performed by ultrafiltration. According to a preferred embodiment, the (clarified) biological sample (e.g., the supernatant) is subjected to an ultrafiltration, preferably a tangential ultrafiltration. Tangential ultrafiltration consists of concentrating and fractionating a solution between two compartments (filtrate and retentate), separated by membranes of determined cut-off thresholds. The separation is carried out by applying a flow in the retentate compartment and a transmembrane pressure between this compartment and the filtrate compartment. Different systems may be used to perform the ultrafiltration, such as spiral membranes (Millipore, Amicon), flat membranes or hollow fibres (Amicon, Millipore, Sartorius, Pall, GF, Sepracor). Within the scope of the invention, the use of membranes with a cut-off threshold below 1000 kDa, preferably between 300 kDa and 1000 kDa, or even more preferably between 300 kDa and 500 kDa, is advantageous.

In a specific embodiment, the clarified tissue culture supernatant (e.g., obtained after clarification through a 0.8 µm filter) is concentrated by ultrafiltration through a 500kDa molecular weight cutoff (MWCO) hollow fiber membrane having a lumen diameter of 0.5mm (A/G Technology Inc). This pore size of the cartridge retains dexosomes in the retentate while allowing proteins that are smaller than the pore size to pass through the membrane. The hollow fiber cartridge contains sufficient surface area to allow the concentration to proceed rapidly without an excess of shear forces on the dexosomes. Typical volume of the starting supernatant that undergoes ultrafiltration is 2 - 4L, which is then reduced to approximately 100mL (20- to 40-fold reduction). The operation setup is depicted in Figure 6.

Other pore sizes such as 30kDa, 100kDa, 300kDa, and 750kDa may also be used to concentrate the dexosomes volume. However, the process efficiency and percent recover may be reduced. Furthermore, other ultrafiltration formats such as the "plate and frame" cassettes from companies such as Millipore, Sartorius, and Filtronics may be used. Stirred cells such as those provided by Amicon Inc may also be used to reduce the volume of dexosomes.

The concentration of dexosomes by ultrafiltration by hollow fiber membranes proceeds under low shear forces. The shear force of the feed stream (e.g. flow rate <300mL/min for 0.7 sq. ft. surface area) is less than 2000sec⁻¹. The inlet and outlet pressures of the system are between 3-8psi during the process. More stringent conditions may be used to concentrate dexosomes compared to the parameters currently used.

Other techniques such as ion exchange and affinity chromatography, as well as flow field-flow fractionation may also be used to concentrate dexosomes in the preparation method of this invention.

### Diafiltration

Diafiltration of concentrated dexosome preparation can be employed to reduce the concentration of contaminating media and cellular proteins. Diafiltration may be performed according to several techniques allowing to exchange the sample buffer with formulation buffer, including ultrafiltration, chromatography, ultracentrifugation, and via a dialysis bag.

In a preferred embodiment, diafiltration is performed with an ultrafiltration system. As demonstrated in the experimental section, this embodiment is efficient. Furthermore, where the vesicle preparation has been concentrated by ultrafiltration, the diafiltration step may be combined easily therewith, using the same methodology.

In this respect, in a particular embodiment, the exosomes are diafiltered by ultrafiltration using the same ultrafiltration membrane (i.e. 500kDa MWCO hollow fiber membrane) as used in the concentration step. This embodiment is advantageous since both steps can be performed essentially in the same device with limited intervention and manipulation of the exosomes, i.e., by mere modification of the products introduced into the hollow fiber.

Besides the 500kDa hollow fiber membrane, other pore sizes such as 30kDa, 100kDa, 300kDa, and 750kDa, can be utilized, preferably comprised between 30 and 1000kDa, more preferably between 200 and 750kDa. The diafiltration buffer may be composed of excipients other than those found in PBS. The volume of buffer used in diafiltration may be anywhere from 1 to 10 times the volume of the dexosome concentrate.

Operation parameters used for diafiltration are similar to those described previously for the concentration of dexosomes from clarified tissue culture media.

### Density Cushion Separation and Purification of exosomes

As indicated, the present invention discloses the treatment of a biological sample comprising membrane vesicles by centrifugation on density cushion, and the recovery of the purified vesicles from the cushion. Density cushion centrifugation provides several advantages over prior art techniques using serial centrifugation or gradient density centrifugation. These include, lack of aggregation of the vesicles which are thus subjected to reduced physical injury, further purification of the vesicles, since additional contaminants can be removed, as will be discussed below, lack of toxicity of the components used in the cushion, reduced sucrose concentration, etc. In particular, the density cushion allows to prevent pelleting of exosomes as the sample is ultracentrifuged. The difference in partial specific volume of soluble protein (about 0.73), exosomes (about 0.88) and protein aggregates makes this an ideal method for separation and purification. Protein aggregates (density about 1.35) are expected to pellet through the density cushion as the exosomes are retained in the density cushion. Briefly, the method is described as follows (a schematic of this process is depicted in Figure 8a and 8b).

The addition of a higher density solution (i.e. cushion) to the bottom of the centrifuge tube compared to the concentrated dexosome solution results in the formation of a discontinuous or step gradient (i.e. sharp changes in density). The cushion results in exclusion of molecules of lower sedimentation coefficients. Furthermore, the cushion makes it easier to resuspend any sedimented material at the conclusion of the run , and prevents damage to particles that may not withstand pelleting.

Moreover, as another advantage, it is believed that the density cushion allows to further eliminate any potential contaminating protein aggregate, in particular haptoglobin aggregates, from the composition.

Membrane vesicles, in particular dexosomes, have been shown to have a density of 1.100 to 1.140 g/mL. Accordingly, the density cushion should have a final density of between about 1.10 and about 1.15.

The composition of the cushion may be adapted by the skilled person, so as to reach the above preferred final density. The cushion solution is preferably slightly hyperosmotic, with osmolarity between 700-800mOs. In a preferred embodiment, the density cushion consists of sucrose/D₂O in Tris buffer. The addition of the dense sucrose/Tris D₂O buffer into the bottom of each tube displaces the concentrated dexosome solution upwards forming a visible interface. Ultracentrifugation at 100,000 x g for approximately one and one-half hour sediments the dexosomes into the more dense sucrose/Tris D₂O cushion. The dense cushion contains an enriched and more purified dexosomes population. Very few dexosomes are either above the cushion or in the pellet that sometimes is visible at the bottom of the tube. Furthermore, D₂O has previously been used in the clinical setting and has not shown any toxicity effects in the concentrations that are being used. In this regard, the natural abundance of deuterium in humans is 15 mg/kg (0.15% wt).

Preferably, where a D₂O/sucrose cushion is used, the density of the initial cushion solution should range between about 1.175 - 1.210 g/mL (deviation from these specific limits can be used as well). Indeed, Applicants now have evidence that diffusion of D₂O/sucrose occurs in the cushion during centrifugation, leading to the formation of a mini-gradient and a final density between about 1.1 and about 1.15 g/ml (i.e. the density of the pooled cushion was measured to be between 1.100 and 1.150 g/mL). This was not expected and provides further advantages to the purification step since, by forming a mini-gradient, the cushion allows further purification of exosomes.

In other instance, other solutions besides sucrose/D₂O may be prepared where the density of the solution is greater than that of the exosomes. In this regard, doubly-labelled water (i.e., D₂¹⁷O or D₂¹⁸O) may be used, that would even allow to further reduce the amount of sucrose in the cushion. Density of available D₂¹⁸O (97% pure) is 1.22. Mixture of D₂O and D₂¹⁷O or D₂¹⁸O could be used to isolate vesicles and cellular fractions of various density without added component.

Higher concentrations of sucrose in H₂O (non-deuterated) may also be prepared to generate a solution of comparable density, although such a solution would be significantly more hyperosmotic and may induce lysis of dexosomes. Commercial density media composed of iodixanol (i.e. OptiPrep), Percoll, and Ficoll may be used to capture dexosomes in a similar fashion, for laboratory process and analytical purpose.

The purified exosomes can be collected from the cushion by any appropriate means, including piercing the tube with a needle, pipetting and the like.

### Formulation and conditioning

### Formulation of Exosomes by Diafiltration

Purified exosomes can be formulated in various buffer or suspension suitable for clinica use or further storage.

For that purpose, purified exosomes can undergo buffer exchange by diafiltration with a 500 kDa ultrafiltration hollow fiber cartridge identical in format to that used previously for concentration and/or diafiltration (see previous discussion). The cartridge is preferably pre-conditioned with a buffer containing hSA (e.g. 100µg/mL) that is void of haptoglobin contamination for a minimum of 15 min prior to diafiltration. It appears that the presence of hSA prevents non-specific loss of dexosomes due to binding to the hollow fiber substrate. The ultracentrifuge cushion fractions (approximately 16mL) containing purified (antigen pulsed) exosomes undergo a minimum of 5 volume buffer exchange to remove the cushion components (i.e. a minimum of 98% buffer exchange). An identical setup to that depicted in Figure 5 is used, however the hollow fiber cartridge is sized down to accommodate the smaller volume. As described previously, diafiltration is performed with low shear forces (i.e. 2000sec⁻¹) which may not be necessary.

A typical SDS-PAGE of a dexosome sample at 1/2800^{th} its original volume is depicted in Figure 10.

Several formulation buffers can be used to formulate exosomes. A typical buffer contains USP/NF excipients. The formulation solution may contain the following components: 1) a buffering agent such as Tris, 2) a cryoprotectant such as sucrose, trealose, glucose, glycerol, etc., 3) salts such as NaCl, KCl, MgCl₂, CaCl₂, etc., 4) bulking agents such as mannitol, glycine, starch, etc., 5) antioxidants, 6) vitamins, 7) stabilizing proteins and peptides such as hSA, and 8) other widely accepted excipients used in formulation.

In this regard, an object of the present invention resides in a composition comprising (i) membrane vesicles, (ii) a buffering agent and (iii) a cryoprotectant or a stabilizing compound. The composition preferably further comprises (iv) salts and/or bulking agent and/or antioxidants and/or vitamins.

Typical buffers comprise PBS, 20mM Tris/5% sucrose/1mM MgCl₂ pH 7.4, or 20mM Tris/5% sucrose/1mM MgCl₂ 100ug/mL hSA pH 7.4 and are subjected to ultrafiltration to remove haptoglobin.

Preferably, the formulation solution as described above is essentially free of haptoglobin (or related polymers, as well as particulate bodies). In this regard, the formulation solution (or particular individual components thereof such as the albumin solution) may be subjected to ultrafiltration to eliminate haptoglobin as disclosed before. This final treatment further provides assurance of higher quality of the product.

### Sterile Filtration and Freezing of exosomes

Finally, the material collected (or formulated) may be subjected to further treatment(s) and/or filtration stages, particularly for sterilization purposes. In this regard, exosomes may be sterile filtered through filters with a diameter less than or equal to 0.3 µm. A typical sterilization comprises filtration through a 0.22µm syringe filter (25mm) with minimal loss (see HLA/DR assay results). Filters composed of Millipore's "Durapore" material can been used in sterile filtration. Other filter materials composed of cellulose acetate or polyether sulfone may also been used. The syringe filters are preferably pre-wetted with the formulation buffer containing 100µg/mL hSA (minus haptoglobin) prior to filtering the exosomes. The exosome sample (~16mL) may be sterile filtered either under controlled parameters with a syringe pump or by hand induced pressure.

The purified exosomes (optionally formulated and/or sterilized) may also be frozen and stored at -80°C, or other storage temperatures of -20°C or 4°C. Freezing is preferably performed at a control rate of 1°/min with a "Mr. Frosty" cryo -1°C freezing container (Nalgene Inc). Other methods of slow or rapid freezing in liquid N₂ may also be employed.

### Quality Control

The present disclosure also discloses and provides novel compositions and methods that can be used to characterize membrane vesicle preparations that have been prepared (e.g., purified and/or formulated), or during the preparation process. The compositions and methods can be used to evaluate the quantity of exosomes in a sample or a composition, to determine the phenotype of an exosome preparation and to assess the biological activity of an exosome preparation. These methods are particularly suited for characterizing a product for use in clinical applications, i.e., to control the quality and composition of an exosome preparation. These methods are very important for therapeutic purposes since essentially autologous (i.e., patient per patient) vesicle preparations are used, which require individual characterization parameters. These compositions and methods can be used to characterize exosome preparations from various origins (i.e., antigen-presenting cells, tumor cells, etc.), antigen-sensitivity (antigen-loaded, antigen-free, etc.), freshly prepared or stored, from primary cells or immortalized cell lines, etc.

### Dosing of Exosomes

A method was developed to assess the quantity of membrane vesicles present in a sample (or a purified composition). The method involves immune complex formation and detection, using antibodies that recognize cell surface markers present on exosomes.

More specifically, the method of dosing membrane vesicles in a sample according to the present invention comprises (i) adsorbing the sample onto a solid support, (ii) contacting the adsorbed support with a (capture) antibody specific for a cell surface marker of exosomes and (iii) determining (or dosing) the presence of antibody-antigen immune complexes.

More preferably, the antibody specific for a cell surface marker of exosomes is an anti-class II antibody, i.e. an antibody that binds MHC Class II molecules, or an anti-class I antibody. Furthermore, in particular embodiments, at least a second capture antibody is used, in parallel, to further increase the selectivity of the assay with regard to exosomes. For instance, preferred additional capture antibodies are directed against the CD81 cell surface marker or against MHC class I molecules. The use of an and-CD81 antibody is advantageous for characterizing (dosing) dexosomes since CD81 is significantly reduced from dendritic cells, and thus provides a signal that is specific to dexosomes. Other antibodies specific to exosomes may be used in this step, such as anti-CD63 or anti-CD9, for instance.

In a preferred embodiment, the exosomes are thus adsorbed onto a solid support and contacted separately with an anti-class II antibody and with an anti-CD81 antibody.

The antibody-antigen immune complexes formed as a result of the contacting step can be detected according to conventional immunological techniques. Preferably, the complexes are revealed using a labeled revelation antibody that binds the first and/or second capture antibodies. The revelation antibody may be labeled with radioactivity, enzymatic activity, fluorescent label, chemunilescent label, etc. The measure of the complexes correlates with (i.e., provides direct indication of) the quantity of exosomes present in the sample. Where two antibodies are used (e.g., anti-Class II and anti-CD81), an average or a ratio of the complexes detected with each antibody may be performed as a quantification parameter.

The various antibodies to be used in the method can be monoclonal or polyclonal antibodies, either in natural form or modified (e.g., humanized), fragments or derivatives thereof. In typical experiments, the capture antibodies are monoclonal antibodies. In a further typical experiment, the revelation antibody is a labeled monoclonal or polyclonal antibody.

In performing the present method, various solid supports may be used, such as a multi wells plate, in particular a 96-wells plate or any other titration plate.

Furthermore, in performing the current dosing method, it is highly preferred to use purified exosome samples in order to reduce non-specific background signal. For that purpose, the sample is preferably a purified exosome sample, even more preferably a sample subjected to density cushion (ultra)centrifugation. For analytical purpose, a few ml of exosome solution is centrifugated on a D₂O sucrose density cushion of 200µl. The ELISA can be performed directly on the cushion solution. This simple purification step allows enough concentration and purification. The recovery is close to 100%.

In a specific embodiment, ELISA-based analyses have been developed in order to provide a quantitative measure of exosomes in a sample, composition, fluid, etc. Figure 11 a illustrates the measurement of HLA-DR (i.e. MHC II) and Figure 11b illustrates the measurement of CD81 in dexosomes. The HLA/DR assay is both sensitive and functionally relevant to the activity of dexosomes, since HLA/DR is involved in antigen presentation. This assay was chosen as the quantitation assay for dexosomes preparation. The ELISA assay for HLA/DR determination quantifies dexosomes in the final product. Since this assay can also measure HLA/DR on dendritic cells, this assay provides a means for relating HLA/DR dose per dendritic cell and per volume of isolated dexosome.

### Phenotyping of Exosomes

A method was developed to assess the phenotype of membrane vesicles present in a sample (or a purified composition). The method involves immune complex formation and detection, using antibodies that recognize several cell (surface) markers present on (in) exosomes. The method further comprises complexing the exosomes onto solid support such as beads, to allow a sensible and reliable determination of the phenotype of each preparation.

In this regard, disclosed is a method of characterizing vesicle membranes in a preparation, comprising contacting, in parallel, samples of the membrane vesicle preparation with two or more antibodies specific for marker of the vesicles and determining the formation of antigen-antibody immune complexes.

The method is more specifically directed at determining the phenotype of an exosome preparation, i.e., the specific type of cell surface markers expressed by the exosomes in the preparation. Considering the autologous nature of exosome preparations, the phenotype is essential in characterizing the composition of the preparation, both in terms of structure and in terms of potential activity.

More preferably, the method of characterizing the preparation comprises an initial step of binding the exosomes onto solid supports, such as beads. Even more preferably, the exosomes are covalently attached to solid supports such as beads, or through antibody-mediated affinity binding. In a typical embodiment, beads with a diameter of about 1 to 10 µm, more preferably 3 to 5 µm are used and can carry above 1000 exosomes covalently attached thereto or coupled by immunoaffinity, more preferably between 2000 and 5000 exosomes. The exosome-coated beads are then distributed into the wells of a plate and contacted, in parallel, with the selected antibodies.

The antibodies can bind either cell surface markers or internal proteins. Indeed, it is believed that the binding of exosomes to solid supports such as beads renders certain internal proteins available to outside agents such as antibodies.

Preferably, two or more of the antibodies listed in Table 1 below are used :

**Table 1**

| **Antibody** | **Source Pharmingen** |
|---|---|
| anti CD11, more preferably | |
| anti CD11c | 30485x |
| anti CD11b | 30455x |
| anti HLA, more preferably anti HLA abc | 32295x |
| anti CD81 | 33475x |
| anti CD63 | 36585x |
| anti CD58 | 36895x |
| anti CD1a | 34224x |
| anti CD1b | |
| anti CD9 | 30374x |
| anti CD86 | 33409x |
| anti CD82 | 35394x |
| anti CD83 | 36934x |
| anti lactadherin* | - |

| | |
|---|---|
| *: rabbit anti-sera against a lactadherin peptide | |

Preferably, these antibodies are labeled to allow the detection and/or dosing of the immune complexes formed. Label can be radioactivity, chemical, enzymatic, fluorescent, etc. Preferred labels are fluorescent, such as FITC or PE.

More preferably, at least 5 different antibodies are used, even more preferably at least 8 different antibodies. The antibodies are preferably monoclonal.

In a specific embodiment, the immunophenotyping is performed using direct staining method. A small aliquot of purified exosomes is incubated with anti-Class II (e.g., anti-HLA/DR)-coated magnetic beads. The exosomes coupled to anti-HLA-DR coated magnetic beads are then incubated with labeled antibodies, more preferably fluorescent conjugated (e.g., FITC or PE) monoclonal antibodies. Using flow cytometry, four parameters can be measured. Forward scatter, side scatter, and two fluorescence channels. This two-color flow cytometry analysis permits identification of the antigens on the exosomes in a single measurement.

Results obtained for several dexosome preparations are presented on Figure 14. These results clearly demonstrate the efficacy and rapidity of the claimed method for immunophenotyping exosome preparations.

### Function of Exosomes

A further method was developed to assess the biological activity of membrane vesicles present in a sample (or a purified composition). The method involves assessing the activation of CTL lymphocytes from a population of T cells, using a reference antigen, e.g., the super-antigen (e.g., SEE).

Disclosed is a method of characterizing the activity of membrane vesicles, comprising contacting super-antigen-loaded vesicles with T cells in the presence of accessory cells, and determining the activation of the T cells.

Superantigens are produced by many different pathogens like bacteria and viruses. Superantigens bind to MHC II molecules directly without being processed. Instead of binding in the groove of the MHC II molecules, superantigens bind to outer surface of MHC II molecules and V_{β} region of T cell receptors (TCR) and are able to stimulate very large numbers of T cells (2-20%). The fact that superantigens can bind to various MHC II and TCR, and induce a strong T cell response make them attractive agents for establishing a general and sensitive assay to test antigen presentation function of dexosomes. Superantigen may be prepared or isolated from various sources. A preferred superantigen for use in the current invention is the SEE antigen, ET 404 (Toxin Technology Inc.). Additional superantigens may be used such as SEA and SEB.

T cells may be any freshly prepared cell population comprising T cells, such as peripheral blood mononuclear cells ("PBMC") for instance, as well as any immortalized T cell line, such as the Jurkat cells. The Jurkat cells are immortalized human T cells, secreting IL-2 upon activation, which can function as the responder cells in this bioassay. The V_{β} 8 of TCR of Jurkat cell binds to SEE. As immortal tumor cells, the Jurkat cells are particularly useful since they are much less heterogeneous and easier to grow in the laboratory than primary cells.

Accessory cells may be any cell capable of mediating the activation signal to T cells in the present bioassay. Accessory cells may be dendritic cells, such as any competent primary dendritic cell culture or dendritic cell line. The accessory cells may also be other immune cells or cell lines, in particular antigen presenting cells or cell lines, such as for Raji cells. Raji cells are EBV-immortalized B-cells and have been shown to function in the present bioassay. Raji cells may be cultured in any suitable medium, such as RPMI for instance.

In carrying out the claimed method, superantigen loaded vesicles are contacted with responder T cells in the presence of accessory cells, and T cell activation is assessed.

Measuring the activation of T cells can be performed according to various techniques, such as cytokine release, protein synthesis, responder cell lysis, etc. In a preferred embodiment, T cell activation is measured by determining the cytokine production in the medium, more specifically interleukin-2 production in the medium. Typically, I1-2 is measured by ELISA.

In the method, the membrane vesicles themselves can be loaded with the super-antigen, or the membrane vesicle producing cells (the resulting vesicles then exposing the super-antigen). In a preferred embodiment, the vesicles are contacted with the superantigen.

In a specific embodiment, in this functional assay, superantigen SEE is first incubated with exosomes prepared from cell culture supernatant by ultrafiltration, cushion density centrifugation, and diafiltration in formulation buffer. Isolated exosomes may be used fresh or can also be stored frozen at -80 degrees in PBS. The complexes of exosome and SEE are then separated from unbound SEE by analytical zonal centrifugation using for instance Optiprep. Optiprep (also known as iodixanol) is a iodinated density gradient media (Nycomed) which allows for a quantitative recovery of exosome/SEE complexes from free SEE. This step is thus important in the biological assay, for quantitative analysis of each prepared exosome lot. The isolated complexes are used to induce T cell activation in the presence of Raji cells as accessory cells. The readout for T cell activation is IL-2 secretion by Jurkat cells.

Particular advantages of the present preparation method over prior art sedimentation techniques using serial centrifugation steps are as follows:
Length of Process Time : Processing by sedimentation is more time consuming in comparison to ultrafiltration and ultracentrifugation. 4L of tissue culture supernatant would require a minimum of 12 hours to process by sedimentation while a combination of ultrafiltration and a single ultracentrifugation run would take 6-7 hours as it currently run.
Closed System - GMP Compliance : The only centrifugation step in the process is performed on a small volume and can be realized in presently available sealed tubes (capacity of a rotor 6 tubes of 33ml each). This eliminates the problem, which would have been encountered if a centrifugation step on a large volume (several litres) had to be performed. No sealed centrifugation tubes exist presently for such large volume. Centrifugation should have been done in non disposable open tubes and would not have complied with present regulatory constraints. The clarification, ultrafiltration and sterile filtration are all performed in a biosafety cabinet, and is considered essentially a closed system. Ultracentrifugation is now also performed with sealed tubes, which eliminates the problems that might arise from contamination in an open tube.
Ultrafiltration of Media : Reprocessing of media, such as AIM V, to remove protein aggregates is an important step in the overall process purification scheme. The upstream removal of co-purifying proteins results in a purer exosome product after processing. In particular, it is estimated that the invention allows to produce compositions that are essentially free of aggregates, i.e., wherein aggregates represent less than 2-4% total protein. Furthermore, the removal of haptoglobin reduced undesirable immune responses.
Potential Aggregation of Exosomes After Sedimentation : Sedimentation of exosomes results in a very high local concentration. The high concentration of exosomes in the pellet has the potential to result in an aggregated product. Electron microscopy gives some indication of exosomes being aggregated after sedimentation compared to the method utilizing ultrafiltration. Secondly, it appears that the ultrafiltration of exosomes results in less debris as visualized by electron microscopy.
Process temperature : The process, as of diafiltration, may be performed on ice, i.e., at a temperature of between about 4 to 10°C. This is advantageous since the equilibrium between free peptides and those bound to exosomes through MHCI can be affected by temperature and since such temperatures prevent action of hydrolytic contaminating enzymes such as proteases..
Exosome Purity : Sedimentation of exosomes runs the risk of contamination with cell debris and media contaminants. The removal of contaminant protein aggregates is particularly important because of their high immunogenicity..
Exosome Recovery : The final recovery is 60-75%, significantly greater than the initial sedimentation method (15% average of 9 experiments). This means that the available dose for the treatment of a patient could be increased 5 fold if required or allow to perform apheresis on a smaller volume of blood and cultivate 5 times less cells causing a considerable lowering of the cost of the process.

Additional aspects and advantages of the present disclosure will be apparent from the following examples.

### EXAMPLES

### 1. Preparation of media

Prior to use, the culture medium (in this example, clinical grade AIM V, a serum-free cell culture medium of Life Technologies, Inc.) has been treated by ultrafiltration to remove aggregated proteins, whose removal does not affect cell growth, but aids considerably in the subsequent isolation of pure dexosomes.

Ultrafiltration of the media was performed using a 500 kDa hollow fiber membrane (from A/G Technology, Needham, Mass or from a vendor carrying a related product). This membrane size retained the protein aggregates in the retentate while allowing the un-aggregated proteins to pass through with the permeate. The permeate is collected, sterile filtered through 0.22 µm filter into bottles, and stored at 4°C until use.

In a specific experiment, to process 50L of AIM V an UFP-500-C-35A (0.5mm lumen diameter, 14.5 sq.ft. surface area) hollow fiber cartridge from A/G technology is used. The feed stream has a flow rate of 13L/min resulting in a permeate flux of 1L/min and an inlet and outlet pressure of 12-16psi and 10psi, respectively. The process is completed within 1 hour. The permeate is sterile filtered through a 0.22µm SartoPore or SartoBran filter (Sartorius Inc). Figures 3 and 4 demonstrate the reduced aggregate content of the medium. Quantitation of haptoglobin by ELISA indicates that greater than 99% of haptoglobin related aggregates are removed by ultrafiltration. More particularly, the UF media contains less than about 5 ng/ml of haptoglobin aggregates.

In particular, the BCA assay indicates that ultrafiltration of AIM V media removes 2-4 % of the total protein in the media. The majority of the fraction of protein removed are aggregates. The protein concentration in the media does not significantly change with the ultrafiltration process, only the concentration of particulates. Pelleting of the ultrafiltered AIM V (UF AIM V) results in any protein pattern substantially different from that observed with un-ultrafiltered AIM V media (see Figure 3). Furthermore, pelleting of ultrafiltered AIM V 3 months post-processing does not indicate the presence of protein aggregates as indicated by SDS-PAGE (see Figure 4), confirming that the treated medium can be stored for long periods of time.

Ultrafiltration of albumin (e.g., hSA) compositions also allows to prepare heated hSA products containing less than about 10 ng haptoglobin aggregates per mg of hSA, i.e., less than about 0.001 % haptoglobin aggregates (wt).

### 2. Immature dendritic cell production and culture

Dendritic cell precursors are harvested from a patient's peripheral blood following leukopheresis. The cell culture procedure is serum free and occurs in clinical grade cell culture media that is further ultrafiltered to remove protein aggregates (or particulate bodies), as described in Example 1. A typical incoming leukopheresis contains about 1 to 2 x E10 cells. The cells are washed four times in PBS supplemented with 0.1% human serum albumin (clinical grade) to remove platelets. The cells are then plated into approximately 100-150 cm² T-flasks at a cell density of 200 x 10⁶ cells/flask in serum free ultrafiltered media. The purification of dendritic cell precursors from the leukocytes in the incoming leukopheresis relies on the adhesive properties of monocytes to charged polystyrene surfaces, such as is present in standard commercial tissue culture flasks. After two hours incubation, the monocytes become adherent and are retained while remaining non-adherent cells are discarded with media exchanges using media supplemented with GM-CSF and IL-4 or IL-13 each at 50 ng/mL, as well as gamma interferon. The adherent monocytes undergo differentiation in the presence of GM-CSF and IL-4 or IL-13 to become immature dendritic cells. On Day 5 of culture, these cells are replenished with more GM-CSF and IL-13 or IL-4. Interferon gamma at 500 U/mL may be added to the cells, in order to maintain dendritic cells in an immature status.

### 3. Procedure for Antigen Loading in a Model System

Preliminary experiments were done to assess the technical parameters for loading antigens into dendritic cells. For this purpose, immature dendritic cells were pulsed with a CMV peptide in order to produce peptide loaded dexosomes. Dexosomes were then isolated by standard procedures and their activity assayed by measuring IFN-γ release by a CMV-specific T cell clone. Dexosomes loaded with CMV peptide specifically stimulated the anti-CMV T cell clone and required the presence of dendritic cells, consistent with our findings using the SEE based activity assay (Figure 2).

Thus using the CMV model system, peptide can be incorporated into dexosomes by the addition of peptide to DC culture on Day 5. Further, our finding that peptide loaded dexosomes require DC for their stimulatory effect on T cells is consistent with the requirement for the inclusion of T cells, DC and dexosomes in the SEE bioassay, thereby underlining the tight correlation between the behavior of dexosomes in these two assays.

### 4. Clarification

4L of tissue culture supernatant is harvested and filtered through a 3/0.8µm Sartoclean CA (500cm² surface area) (Sartorius Inc) at 250mL/min. The inlet pressure on the filter does not exceed 10psi (Figure 5).

### 5. Concentration

4L of clarified tissue culture media was concentrated to 100mL with an UFP-500-C-4A hollow fiber cartridge (0.7 sq. ft. surface area; 0.5mm lumen diameter) from A/G technology. The feed stream flow rate was between 225-275mL/min and the inlet and outlet pressure 4-7 psi and 3-6 psi, respectively. The permeate flow rate is between 40-60mL/min under these conditions. The process required approximately 60-80min to complete (Figure 6).

### 6. Diafiltration

In a specific example, the exosome concentrate was diafiltered against 5 volumes of PBS (i.e. 100mL dexosome concentrate diafiltered against 500mL of PBS). A SDS-PAGE of concentrated dexosomes before and after diafiltration is depicted in Figure 7. This step, as well as all subsequent steps were performed at chilled conditions (between about 4-10 °C).

### 7. Density Cushion Separation (Discontinuous Gradient)

In a specific example, the concentrated and diafiltered culture medium containing exosomes, as well as those examples where the exosome have been pulsed with antigen(s), is purified by (ultra)centrifugation on a density cushion as follows: The exosome concentrate (approximately 100mL) is equally aliquoted into centrifuge tubes and underlayed with 4 mL of a sucrose/Tris D₂O buffer. The D₂O density cushion consists of 25-30% sucrose/20mM Tris D₂O (w/w%) (pH 7.5 - 7.7). The density is between 1.18 and 1.21 g/ml. The tubes are sealed to ensure a closed system.

Figure 9a depicts a SDS-PAGE of the sample before and after ultracentrifugation as well as the content above the cushion, in the cushion, or in the pellet. As shown, the majority of the protein does not sediment into the sucrose/Tris D₂O density cushion. This is further confirmed on Figure 9b, which demonstrates that at least 90% of the exosomes are recovered in the cushion.

### 8. Formulation and conditioning

The purified exosomes were subjected to buffer exchange by diafiltration with a 500 kDa ultrafiltration hollow fiber cartridge identical in format to that used previously for concentration and/or diafiltration (see examples 5 and 6). The cartridge was pre-conditioned with hSA (e.g. 100ug/mL) for a minimum of 15 min prior to diafiltration and sized for the processing of 10-20mL of media. A typical SDS-PAGE of a dexosome sample at 1/2800^{th} its original volume is depicted in Figure 10.

The formulated exosomes were sterile filtrated through 0.22µm syringe filter (Millex GV (25 mm syringe)).

The amount of haptoglobin aggregates present in the exosome preparation (200-fold concentration from original media) conditioned in a PBS buffer was measured by ELISA and determined to be essentially below about 0.1 ng/ml, more specifically 0.091 ng/ml or 0.044 ng/ml for the two preparations tested. In comparison, the content of aggregated haptoglobin in exosome preparation obtained according to prior published methods, after an identical 200-fold concentration, was found by ELISA to be 400 µg/ml. This corresponds to a quantitative recovery of the haptoglobin aggregates present in the unpurified AIMV medium (2 µg/ml). Thus, the method of this invention, as compared to the prior art methods, leads to a 5 to 10 millions fold decrease of haptoglobin aggregates contamination in exosome preparations (400 µg/ml versus 40 - 90 pg/ml). Since ultrafiltration of the media reduces the content of haptoglobin aggregates by a factor of about five hundreds, the additional ultrafiltration, diafiltration and/or density cushion steps also contribute very significantly to the increased purification performance. These results further illustrate the efficacy of the present methods in removing haptoglobin aggregates.

### 9. Dosing of exosomes

In a specific embodiment, ELISA-based analyses have been developed in order to provide a quantitative measure of exosomes in a sample, composition, fluid, etc. Figure 11 illustrates the measurement of HLA-DR (i.e. MHC II) (11b) and CD81 (11a) in dexosomes. The HLA/DR assay is both sensitive and functionally relevant to the activity of dexosomes. This assay was chosen as the quantitation assay for dexosomes preparation. The assay is described in detail below.

The HLA/DR signal measured by ELISA is used to determine the number of MHC II molecules obtained from a dexosome preparation. The number of MHC II associated with a number of cell types has been reported previously (Cella et al 1997). These values are summarized in Table 2.

**Table 2 Approximate numbers of MHC II molecules per cell number (adapted from Cella et al 1997)**

| Cell Type | MHC II molecules /cell (x E6) |
|---|---|
| Fresh monocytes | <0.1 |
| Cultured monocytes | <1.0 |
| Raji cells | 2.0 |
| Immature DC | 5.2 |
| Mature DC | 8.3 |

To quantitate the amount of HLA/DR molecules, DC lysate or Raji cell lysate is used as standards. Figure 11 shows a titration of anti-HLA/DR at a fix concentration of immature DC lysate. From this plot, the number of HLA/DR molecules to immature dendritic cells is calculated to be 5.8 x 10⁶ molecules/cell, which is in agreement with the literature value presented in Table 1. An identical result was obtained for Raji cell lysate (data not shown).

An example of the HLA/DR assay results obtained from a dexosome preparation is depicted in Figure 12(A,B). The HLA/DR signal is plotted as a function of the equivalent supernatant volume. The number of HLA/DR molecules/uL dexosome was determined to be 4.7 x 10¹⁰ after ultracentrifugation onto a density cushion (i.e. UC-cushion) and diafiltration into formulation buffer. The % recovery for that sample was 73% HLA/DR. More particularly, the % recovery was as follows :

| | |
|---|---|
| Process Step | Overall % Recovery |
| Clarification through 0.8 µm | 100% |
| 1^{st} Ultrafiltration - Concentration | 100% |
| 1^{st} Ultrafiltration - Diafiltration | 86+/-2% |
| Cushion - 2^{nd} Ultrafiltration - Diafiltration | 85+/-2% |
| 0.2µm Sterile Filtration | 75+/-3% |

In summary, the ELISA assay for HLA/DR determination quantifies dexosomes in the final product. Since this assay can also measure HLA/DR on dendritic cells, this assay provides a means for relating HLA/DR dose per dendritic cell and per volume of isolated dexosome. In this regard, we are generally able to generate a minimum of 100,000 HLA/DR molecules per immature DC after purification (10-12 experiments).

A similar assay was performed using an anti-class I antibody for capture, namely the antibody produced by HC-10 cells. The results are presented on Figures 12(C,D). From the titration curve (12C), the amount of MHC-I molecules can be determined from any unknown exosome preparation (12D).

### 10. Phenotyping of Exosomes

5 to 10µl of anti-HLA-DR-coated magnetic beads are washed in a microfuge tube with 500 µl of PBS using a magnetic rack. The supernatant is discarded and 25, 50 or 100µl of concentrated exosome preparation are added to the washed beads (exosome concentration may be up to 1000 times). The mixture is incubated at 4°C for about 2 hours on a rotating plate. After incubation, 500 µl PBS are added to the exosome-coupled beads and the beads are washed using the magnetic rack. The supernatant is removed and the exosome-coupled beads are suspended in 200 µl of staining buffer. 20 µl of the exosome-coupled beads solution are aliquoted in test tubes which are then contacted each separately with one of the labeled antibodies selected for analysis. The antibodies are incubated for about 30 minutes at 4°C. The tubes are then added with staining buffer and centrifuged at 1200 rpm for 5 minutes. The supernatant is discarded and a fixative solution is added (0.5 ml) in each tube. Each assay tube is then acquired and analyzed by flow cytometry using a FACSCalibur^{™} apparatus.

Results obtained for several dexosome preparations are presented on Figure 13. These results clearly demonstrate the efficacy and rapidity of the claimed method for immunophenotyping exosome preparations.

### 11. Functional Assay

Approximately 20 µl of isolated dexosomes are needed for each test. Dexosomes are incubated with SEE at 100 ng/ml (10 µl of a stock of 1µg/ml SEE in PBS with 0.1% BSA) in the final volume of 100 µl for 1 hour at 37 degrees. After the 1 hour incubation, complexes of dexosome and SEE (dexosome/SEE) are separated from unbound SEE by zonal ultracentrifugation (2.2mL) onto a two step discontinuous gradient composed of Optiprep solutions (Figures 14-16).

The Optiprep gradient is made by first adding 1.7 ml of 10% Optiprep (1.67 ml Optiprep plus 8.33 ml RPMI) into each test tube. Next, 100 µl of 20% Optiprep (3.33 ml Optiprep plus 6.67ml RPMI) are added to the bottom of each tube, followed by addition of 100 µl of 40% Optiprep (6.67 ml Optiprep plus 3.33 ml RPMI) below the 20% Optiprep. Exosome/SEE samples are then layered onto the top of the gradient in a final volume of 200 µl in 0.1% BSA in PBS. The tubes are centrifuged for 40 minutes at 100,000 rpm at 4 degrees using slow acceleration and deceleration in a swinging bucket rotor (TLS-55). 200 µl of samples are collected from the bottom of the tube, which contains the complexes of exosome/SEE freed of unbound SEE. Free SEE remains in the zonal layer (10% Optiprep), while the exosomes/SEE complex sediments into the 20-40% region of the tube. Because optiprep was found to interfere with the ELISA determination, the use of D20 sucrose gradient is preferred if such determination is required

Raji cells, dexosome/SEE (or mock/SEE), and then Jurkat cells are added into wells of a plate (100 µl (30,000 cells) of each kind of cells is used for each well). Positive and negative controls are also added : Jurkat cells alone (negative control), Jurkat plus 1 ng/well SEE (negative control), Raji alone (negative control), Raji and Jurkat plus dexosomes un-pulsed with SEE (background control), Raji and Jurkat plus 1 ng/well SEE (positive control). The plate is incubated for 18 hours 37 degrees with 5% CO2. The cell culture supernatant is collected from each of the wells of the plate by centrifugation at 1200 rpm (use plate carriers) for 15 minutes. 200 µl of the supernatant are used in an ELISA assay to measure IL-2 secretion. The ELISA assay may be performed immediately or later (in which case the plate can be wrapped in parafilm and frozen until use).

The IL2 ELISA assay is performed using the IL-2 ELISA kit (Duo set DY202, R&D Systems) in Costar ELISA plates (Costar 2581), following instructions of the manufacturer The results show a dose-dependent relationship between IL2 secretion and the amount of exosomes per well, which has been converted to the volume equivalent to that of exosome stock. IL2 secretion is SEE-dependent, because exosomes require SEE to stimulate non antigen-specific T cells (e.g., Jurkat). The unit of half max is introduced as a semi-quantitative measurement for dexosome's titre in the method. It is defined as the volume of exosome at which the half maximum IL2 secretion by Jurkat cells is reached under the used experimental conditions. A smaller half-max unit measured is indicative of a higher titre.

### 12. Direct Peptide Loading

This example describes the methodology of direct loading, parameters affecting the loading, competition of the binding between reference peptide and target peptides to dexosomes, and bioactivity of the dexosomes loaded with antigen. A protocol based on HLA-A2 restricted peptide direct loading is generated. The same protocol can be applied for HLA-A1 restricted peptides. Significance and advantages of direct peptide loading of dexosome in tumor therapy clinical trials is also discussed.

This example shows that it is possible to load antigenic molecules directly to dexosomes, and that such direct loaded dexosomes are able to stimulate CTL clones. The results show that direct loading may be more efficient than indirect DC loading, since lower amounts of peptides are necessary.

### 12.1. Methods

### Direct loading peptide to exosome in the presence and absence of β2-m

HLA-A2 restricted reference peptide FLPSDCFPSV, derived from a natural epitope of Hepatitis B core antigen, is biotinylated via cysteine. It competes with unlabeled reference peptide, suggesting the biotinylated peptide maintains its ability to bind to HLA-A2 molecules. For direct peptide loading in the presence of exogenous β2-m, 100 µl of purified HLA-A2⁺ and HLA-A2⁻ negative dexosomes are treated with an equal volume of citric acid or acetate buffer, pH 3.2-5.2 at 4°C for 90 seconds, lowering the pH to favor elution of bound endogenous peptides. After the treatment, the preparation is immediately neutralized with a cocktail of pH 11 containing Tris, exogenous peptide (from 0.01 to 10 µg/ml final concentration) and β2-m (from 0-80 µg/ml final concentration), and incubated at room temperature to allow reformation of the tri-molecule complex of class I, β2-m, and peptide on the dexosome surface. For direct loading in the absence of exogenous β2-m, dexosomes are mixed with exogenous peptide (10 or 100 µg/ml final concentration) first then with an equal volume of acetate buffer, pH 4.2 -5.2, and incubated at room temperature for 30 minutes. During this period, the exchange of peptides occurs resulting in the binding of the exogenous peptide present in excess. The exogenous peptide can be biotinylated peptide alone or a mixture of the labeled peptide with increasing amount of a target peptide. Removal of unbound peptide and β2-m is not necessary since the signal source is from Class I/peptide complexes captured by anti-HLA-Class I antibodies described as below.

### Measurement of reference peptide bound to dexosome with TRF

Peptide loaded dexosomes are lysed by 1% NP 40 on ice for 30 minutes. Complexes of Class I molecules containing labeled reference peptide in the lysates are captured by an anti-Class I antibody via rabbit anti-mouse IgG coated on an ELISA plate. After incubation and washing, Europium labeled streptavidin is added and fluorescence signal from Europium is generated by the Enhancement Buffer and read out with the Victor 2 Fluorescence Reader according to the manufacturer's instructions (Wallac). Comparing the fluorescence signal level with that of a quantitative Europium standard, the absolute amount of the biotinylated reference peptide can be estimated based on the specific activity of Europium labeled streptavidin.

### Competition of the binding to dexosome of reference peptide with target peptides

### Competition in the presence of β2-m

Up to 100 fold excess of MAGE 3A1, MAGE-3, 4, and 10 peptides were mixed with 5 µg/ml of biotinylated reference peptide in loading buffer containing 20 µg/ml of β2-m, and loaded onto acid treated dexosomes. The signal of reference peptide is detected by TRF and reduction of the signal by MAGE 3, 4, and 10 is calculated.

### Competition in the absence of β2-m

Unlabeled peptide in 5-20 fold excess of biotin-reference peptide (100 µg/ml) was prepared in acetate buffer of pH 4.8 or 5.2. This peptide was added to an equal volume of dexosome and incubated at room temperature for 1 hr. The signal of reference peptide is detected by TRF and the reductions in signal by MAGE-3, 4, and 10, or MAGE-3A1 are calculated.

### SEE assay

One hundred µl of peptide direct loaded dexosomes in the presence and absence of β2-m are incubated with 100 ng/ml SEE at 37°C for 1 hr. After removing unbound SEE by zonal density gradient centrifugation, the complexes of dexosome and SEE are incubated with Raji cells and Jurkat cells for 18 hrs. Culture supernatant is collected to measure IFN-γ secretion from Jurkat cells.

### Mart-1 specific CTL clone stimulation assay

Dexosomes directly loaded with MART-1 peptide as described above are washed of free peptide after the loading, and incubated with DC and T cell clone LT 11 cells for 24 hours. Biological activity is measured by IL-2 secretion with an ELISA according to the following T cell clone assay.

The peptide used is an HLA-A2 restricted MART-1 peptide having the sequence ELAGIGILTV.

Unbound Mart-1 peptide is removed by density gradient centrifugation. It should be understood that the removal of unbound peptide from dexosome is not mandatory, but reduces unspecific binding.

T Cell Clone Assay

20x 10³ HLA A2 positive or negative monocyte derived dendritic cells (BM-DC) were incubated for 2 hours with 15 µl (1x10¹⁰ class ii) of the dexosome preparation in U-bottom 96-well plate, at 37°C, then, 20x 10³ cells of the T cell clone LT11 were added to the Human BM-DC cells. the final volume per well was of 200µl. 24 hours later, supernatants were harvested and analysed for IL-2 presence by ELISA. HLA A2⁺ BM-DC cells pulsed directly with 10µm of mart peptide (ELAGIGILTV) were used as positive control.

### P1A Peptide Loaded Dexosomes.

Murine H2^{b} and H2^{d} dexosomes were directly loaded with the OVA peptide 257-269 (SIINFEKL) as follows : 1 volume of Na acetate buffer (0.2M, pH5) was added to 1 volume of exosomes and incubated, for 90 seconds, at 4°C, then neutralized by addition of tris 2M solution (pH 11) containing 10 µm of OVA peptide and 40 µg/ml of human b2-microglobulin. after an incubation of 4h at room temperature OVA dexosome complexes were separated from unbound OVA peptide by an optiprep gradient.

### T Cell Clone Assay

25 X 10³ D1 cells were incubated for 30 minutes with 25 µl or 50µl of the dexosome preparation in U-bottom 96-well plate, at 37°C. then, 25 10³ cells of the T cell hybridoma (B3Z ) were added to the D1 cells. the final volume per well was of 200µl. 24 hours later, supernatants were harvested and analysed for IL-2 presence by ELISA. D1 cells pulsed directly with 10µm of OVA peptide were used as positive control.

### 12.2. Results

### Direct loading of HLA-A2⁺ dexosome in the presence of β2-m

The direct peptide loading was first performed with HLA-A2 restricted reference peptide H-FLPSDC(biotin)FPSV-OH on HLA-A2⁺ dexosomes, as described. HLA-A2-dexosomes were used as HLA specificity control. Figure 17 shows HLA-A2 molecules loaded with biotin-labeled reference peptide captured on the plate by anti-Class I antibody after dexosome lysis. The binding of reference peptide to dexosome is HLA-A2 specific because it does not bind to HLA-A2⁻ dexsomes. The binding depends on both peptide and the amount of dexosome.
Parameters for direct peptide loading were assessed using the TRF assay described. Buffer choice was influenced by the effect on peptide loading efficiency and on dexosome integrity as measured by SEE assay. A range of pH of the loading buffers was tested to optimize conditions for loading of reference peptide. We found comparable signals for peptide binding were obtained using similar loading conditions with either citric acid phosphate buffer or sodium acetate buffer (Figure 18). To test whether mild acid treatment might change conformation of the molecules expressed on dexosome and impair their functions, we performed a superantigen assay in which superantigen SEE, bound to HLA-DR molecules of dexosomes, induced IL-2 secretion from Jurkat cells in the presence of Raji cells as accessory cells. Figure 19 shows that dexosome preparations treated with sodium acetate at pH 4.2 induced the same amount of IL-2 secretion as untreated control in the SEE assay. Dexosomes treated with citric acid/phosphate buffer at the same pH 4.2 also induced IL-2, although to a much lower extent. In this regard, citrate buffer may inactivate the exosomes so that conditions used to load whole cells using citrate buffer are not efficient for loading exosomes. The sodium acetate buffer was thus selected for further studies. We next tested whether the binding of peptide to dexosome HLA-A2 is enhanced by the presence of exogenous β2-m. Figure 20 shows that with 10 µg/ml of the HLA-A2 restricted biotin reference peptide, addition of 20 µg/ml of β2-m significantly increases the amount of the reference peptide bound.
In order to determine the amount of peptide necessary to saturate binding to HLA A2 molecules, we performed a peptide titration where 0-20 µg/ml of the peptide is added during the loading. Figure 21 shows the loading is saturated when more than 1.25 µg/ml of reference peptide is used. The kinetics of peptide loading was evaluated using three different dexosome preparations by varying incubation time at room temperature. Figure 22 indicates peptide binding is relatively constant between thirty minutes and 4 hours of incubation among all three dexosome tested, indicating the majority of the binding occurs within 30 minutes. From our studies, we conclude that loading using mild acid elution with pH 4.2 sodium acetate buffer, 10 µg/ml class I peptide, 20 µg/ml β2-m, for a loading time of 30 minutes at room temperature provide the optimal conditions for class I peptide loading onto dexosomes.
We designed peptide competition assays to provide evidence that unlabeled target peptides can be loaded onto dexosomes in the same way. Unlabeled target peptides MAGE-3, 4, or 10 were mixed with 5 µg/ml labeled reference peptide at the ratios of 1 - 100. The reduction of fluorescence signals from reference peptide indicates competitive binding of unlabeled target peptide to dexosomes. As demonstrated in Figure 23, all the three MAGE derived peptides competed reference peptide off binding from dexosomes. MAGE 3 and 10 competed better than MAGE- 4 indicating perhaps higher binding affinity to HLA-A2 molecules than MAGE-4 under the loading conditions:

A functional assay using a MART-1 peptide specific T cell clone, LT11, was established to test the biological activity of dexosomes, which measures antigen specific T cell responses *in vitro.* Following the protocol for direct loading of dexosomes with class I peptides, a MART-1, HLA-A2 restricted peptide was loaded onto HLA-A2⁺ or HLA-A2⁻ dexosomes at the peptide concentrations of 0.01 to 10 µg/ml. Loaded dexosomes were subsequently washed by density gradient centrifugation to remove unbound peptide. The results are presented Figure 24 and show that HLA A2⁺ BM-DC cells pulsed with 10 µM of Mart were able to activate LT11 clone that recognized HLA A2 with the Mart peptide. The HLA A2⁻ BM-DC cells can't stimulate LT11 clone. BM-DC cells pulsed with 15 µl of Mart directly loaded HLA A2⁺ dexosomes were able to activate LT11 clone whatever the haplotype of the dendritic cells.
Previously, indirect peptide loaded dexosomes only induced marginal levels of IL-2 secretion even when 10 µg /ml Mart-1 peptide was added to the DC culture to prepare the dexosome. Dexosomes directly loaded with Mart-1 peptide consistently and reproducibly stimulated the LT 11 clone, suggesting that direct peptide loading is a more efficient way to produce biologically active dexosomes.

The same protocol was applied for direct loading of an HLA-A1 restricted peptide. In the experiment, a biotinylated reference peptide EVDPC(biotin)GHLY, derived from the natural HLA-A1 restricted, MAGE3-A1 peptide EVDPIGHLY, binds to Al⁺/A2- dexosome (Exo 433), but not to A1⁻/A2⁺ dexsome (Exo 427). Conversely, the HLA-A2 restricted, biotinylated reference peptide derived form Hepatitis B core antigen, binds to A1⁻/A2⁺ but not to A1⁺/A2- dexosome, demonstrating a strict HLA-allele-dependent loading of Class I antigen peptides. The bottom panel of Figure 10 shows unlabeled MAGE3-A1 peptide competed with the biotinylated MAGE-3A1 peptide for binding on A1⁺ dexosome (Exo 433).

The direct loading approach was also applied to a PIA peptide and murine dexosomes. The results are presented Figure 25. D1 cells pulsed with 25 µl or 50 µl of OVA directly loaded H2^{b} dexosomes were able to activate B3Z cells that recognizes K^{b} with the OVA peptide. We also observed, to a lower extent, an activation when D1 cells were pulsed with OVA directly loaded H2^{d} dexosomes.

Similar results are obtained using HLA-A1 restricted peptides.

### Direct loading of HLA-A2⁺ dexosome in the absence of β2-m

As shown in Figure 20, after acid elution at pH 4.2, exogenous peptide could be loaded to dexosome at neutral pH without adding exogenous β2-m. Because the signal of binding was one quarter to one third of that in the presence of 20 µg/ml of the β2-m, we optimized experimental conditions to increase loading efficiency in the absence of exogenous β2-m. Two peptide concentrations, 10 and 100 µg /ml of biotinylated reference peptide, and sodium acetate buffers of pH 4.2, 4.5, 4.8, and 5.2 were tested. Figure 26 shows that the loading of reference peptide is greatly enhanced at 100 µg /ml with the acetate buffer of pH 4.8.
Unlike loading dexosomes in the presence of β2-m, in which dexosomes are quickly neutralized after 90 second of acid treatment, loading dexosomes in the absence of β2-m requires they stay at pH 4.8 or 5.2 for prolonged period, typically a minimum 30 minutes at room temperature. Figure 27 shows that there are no differences in IL-2 secretion of Jurkat cells induced by SEE loaded dexosomes, untreated or treated with pH 4.8 or 5.2, indicating that the treatment did not damage the exosomes.
To demonstrate that the target peptides, MAGE-3, 4, and 10 can be loaded in the absence of β2-m, a set of competition experiments was performed. In Figure 28, 5-20 times of MAGE-4 and MAGE-10 peptide competed with the reference peptide at pH 4.8. Inhibition by 20 fold excess of MAGE-4 or MAGE-10 is 35% or 66% respectively, similar to the levels of inhibition in the presence of β2-m at pH 4.2 (See Figure 23) indicating that the two peptides can be loaded by both methods. Figure 29 shows the inhibition by Mage-3 at pH 5.2. Loading MAGE-3 seems more effective at pH 5.2 than at pH 4.8 because the competition by MAGE-3 is much weaker at pH 4.8 (data not shown).
Based on the above results, we loaded Mart-1 peptide to HLA-A2⁺ in the absence of β2-m and tested them in LT 11 assay. Figure 30 shows biological activity of dexosomes loaded with Mart-1 peptide in the absence of β2-m. In this experiment, Mart-1 peptide at 10 and 100 µg/ml was loaded onto HLA-A2⁺ (Exo 447) and HLA-A2⁻ (Exo 450) dexosomes at both pH 4.8 and 5.2 in the absence of β2-m. As control, Exo 447 was also loaded with 10 µg/ml of Mart-1 in the presence of β2-m. Dexosomes loaded at the three different conditions (pH 4.8 without β2-m, pH 5.2 without β2-m, pH 4.2 with β2-m) all had similar biological activity, indicating that β2-m is not absolutely required for generating biologically active dexosomes.

### Direct loading of HLA-A1⁺/B35⁺ dexosome in the presence and absence of β2-m

The same protocol can be applied for direct loading of an HLA-A1 or B35 restricted peptide. The nonameric MAGE-3A1 (EVDPIGHLY), derived from MAGE-3 protein is presented by HLA-A1 and HLA-B35. To test whether this peptide can be loaded on HLA-A1⁺ dexosomes, we designed a reference peptide named MAGE-3A1C5 in which the isoleucine of the MAGE-3A1 peptide is substituted with a cysteine and labeled by biotin. In the experiment shown in top panel of Figure 31, the biotinylated MAGE-3A1C5 was loaded to a HLA-A1^{+/} A2⁻ dexosome (Exo 433) in the presence of β2-m. We found the same peptide could not be loaded to HLA-A1⁻/A2⁺ dexosome (Exo 427). Conversely, the HLA-A2 restricted, biotinylated reference peptide derived from Hepatitis B core antigen, could be loaded to A1⁻/A2⁺ but not to A1⁺/A2⁻ dexosome, demonstrating a strict HLA-allele-dependent loading of Class I antigen peptides. The bottom panel of Figure 31 shows the natural MAGE3-A1 peptide competed with the biotinylated MAGE-3A1C5 peptide for binding on A1⁺ dexosome (Exo 433), indicating the binding capacity of unlabeled MAGE3-A1 peptide to the A1⁺ dexosome
Because of limited source of HLA-A1⁺ dexosome, we used HLA-B35⁺ dexosome (Exo 426) to test the binding of MAGE 3-A1 and inhibition in the absence of β2-m. Using the same loading conditions for HLA-A2⁺ dexosome, we directly show in the top panel of Figure 32 that MAGE-3A1C5 can bind to HLA-B35⁺ dexosome at either pH 4.8 or pH 5.2, with less non-specific binding to HLA-A1⁻B35⁻ dexosome at pH5.2 (Exo 424). The bottom panel of Figure 32 shows the inhibition of MAGE-3A1C5 by natural MAGE-3A1 peptide at pH 4.8 and 5.2 in the absence of β2-m. Binding inhibition by natural MAGE-3A1 was much stronger at pH 4.8, suggesting better binding capacity of the unlabeled MAGE-3A1 peptide to HLA-B35⁺ dexosome at this pH. These results show the broad application of direct peptide loading on other HLA Class I alleles.

This example thus describes a highly efficient method to produce biologically active dexosomes loaded with immunigenic compounds (e.g., class I restricted tumor-derived peptides) via a process known as direct loading. Dexosome class I molecules stripped of endogenous peptides and β2-m by mild acid treatment can be efficiently loaded with the target peptides, as shown by biochemical and functional data. Comparing the fluorescent signals of reference peptide bound to dexosomes with Europium standards, the amount of peptide bound to dexosomes can be quantitatively measured.

Table 3 shows absolute amount of reference peptide bound to dexosomes in the absence of β2-m. There are between 0.1-1.2 ng of reference peptide per 10¹⁴ Class II molecules, depending on the donor and the loading conditions used. This number is strikingly close to that of a manufactured dexosome preparation (1 ng per 10¹⁴ Class II molecules) in which peptide loading at pH 4.8 with 10 µg/ml peptide was performed in a much larger scale. Even though the binding is less in the absence of β2-m (Figure 23), our LT 11 assay data (Figure 30) show similar biological activity between the dexosomes loaded with different conditions, indicating that direct loading in the absence of β2-m is sufficient for biological function.
Quantification of Class I molecule levels on dexosomes by TRF and Elisa permits determination of the occupancy of peptide on Class I molecules. We have estimated that the occupancy of the reference peptide on HLA-A2 molecules of dexosomes reaches up to 15 %, more preferably up to 40%. High peptide occupancy greatly improves T cell stimulating activity, measured by the in vitro T cell assays. Moreover, quantification of peptide loading and determination of the peptide occupancy on Class I molecules on dexosomes will aid in evaluating the dose of dexosome based therapeutic candidates, an important aspect for designing animal studies and future clinical trials.
Direct loading can also be done in the absence of β2-m. Using a high peptide concentration and a mild acid buffer, peptide exchange on HLA molecules was observed. We have demonstrated by competition experiment that our target peptides MAGE-3, 4, and 10 can be specifically loaded to HLA-A2⁺ dexosomes in this manner. Mart-1 peptide loaded dexosome in the absence of β2-m stimulated IFN-γ secretion by LT 11 demonstrating the biological activity of the dexosome. Therefore manufacturing biologically active dexosome without β2-m is feasible thus eliminating the present difficulties of using β2-m purified from human donors or non-GMP grade recombinant β2-m. The overall loading is lower in the absence of β2-m, but it does not seem to affect LT 11 activation, suggesting the bioassay is much more sensitive than biochemical assay.
We have demonstrated that direct peptide loading can have broad applications on various HLA Class I alleles like HLA-A2, A1, and B35. Previously, peptide loading of dexosomes relied on an indirect approach, entailing increased manipulation of the cell culture and thus increasing chances of contamination. Direct peptide loading is safer in dexosome manufacturing by eliminating the need of β2-m and the arduous task of adding peptide into each of the culture flasks.

**TABLE 3**

| **Dexosome Prep** | ***HLA-classll* (10**^{**10**}**/µl)** | **fmol peptide/ 16.6 µl Dex sample** | **µg peptide (10**^{**-9**}**) / 10**^{**14**} **classII** |
|---|---|---|---|
| **pH 5.2 No β2-m** | | | |
| **100 µg/ml pep** | | | |
| Dex 375 | 2.89 | 1.4 +/-0.1 | 0.42 |
| Dex 430 | 3.99 | 6+/-.05 | 1.3 |
| Dex 431 | 1.44 | 1.99 +/-0.1 | 1.2 |
| Dex 447 | 12.7 | 1.52 +/-0.1 | .01 |
| PQ01 | 6.57 | 1.3 +/-0.2 | 0.2 |
| VP0023-02 | 1.21 | 0.93+/-0.1 | 0.7 |
| **pH 5.2 No β2-m** | | | |
| **10 µg/ml peptide** | | | |
| 430 | 3.99 | 0.9 +/-0.2 | 0.2 |
| 447 | 12.7 | 1.6 +/-.06 | 0.11 |
| **pH 4.8 No β2-m** | | | |
| **100 µg/ml peptide** | | | |
| 375 | 2.89 | 4.6 +/-0.4 | 1.4 |
| 447 | 12.7 | 8.8 +/-0.2 | 0.6 |
| PQ01 | 6.57 | 5.9 +/-10 | 0.8 |
| VP0023-02 | 1.21 | 3.6 +/-0.3 | 2.6 |
| **pH 4.8 No β2-m** | | | |
| **10 µg/ml peptide** | | | |
| 375 | 2.89 | 1.2 +/-0.3 | 0.36 |
| 447 | 12.7 | 2.4 | 0.16 |
| PQ01 | 6.57 | 1.1 +/-0.1 | 0.14 |
| VP0023-02 | 1.21 | 1.7 +/-0.5 | 1.2 |

## Claims

1. A method of preparing an immunogenic membrane vesicle, the method comprising isolating or purifying the membrane vesicle from a biological sample and contacting said purified membrane vesicle with a peptide or a lipid under conditions allowing the peptide or lipid to bind an antigen-presenting molecule at the surface of said membrane vesicle.

2. The method of claim 1, wherein the membrane vesicles are isolated from a biological sample comprising antigen-presenting cells.

3. The method of claim 2, wherein the membrane vesicles are isolated from a biological sample comprising human dendritic cells.

4. The method of claim 1, comprising the step of subjecting the isolated or purified membrane vesicles to a selected acid medium prior to, during, or after contacting said vesicles with said peptide or lipid.

5. The method of claim 1, wherein, after contacting, the vesicles are subjected to density centrifugation or diafiltration to remove unbound peptide or lipid.

6. The method of claim 1, wherein the peptide is a class-I restricted peptide.

7. The method of claim 1, wherein the peptide is a class-II restricted peptide.

8. The method of claim 1, wherein the vesicles are contacted with a mixture of peptides.

9. The method of claim 8 wherein the vesicles are contacted with a peptide eluate of tumor cells.

10. The method of claim 1, wherein the antigen-presenting molecule is a CD1 molecule and the lipid is selected from a microbial lipid, a microbial glycolipid and a lipid or glycolipid tumor antigen.

11. The method of claim 1, for preparing an immunogenic membrane vesicle comprising a complex of an exogenous class-I peptide bound to an HLA class I molecule, the method comprising (i) subjecting an isolated or purified membrane vesicle to a selected acid medium, (ii) contacting said isolated or purified membrane vesicle with a class I-restricted peptide in the presence of beta2-microglobulin, under conditions allowing the peptide to complex with an HLA class I molecule at the surface of said membrane vesicle, and (iii) collecting the loaded membrane vesicle.

12. The method of claim 11, wherein step (i) comprises subjecting the isolated or purified membrane vesicles to a medium at a pH comprised between about 3 and 5.5 for less than 15 minutes.

13. The method of claim 11, wherein step (ii) comprises contacting the isolated or purified membrane vesicle with 0.005 to 50 µg/ml of class I-restricted peptide in the presence of beta2-microglobulin.

14. The method of claim 1, for preparing an immunogenic membrane vesicle comprising a complex of an exogenous class-I peptide bound to an HLA class I molecule, the method comprising (i) contacting an isolated or purified membrane vesicle with a class I-restricted peptide in the absence of beta2-microglobulin, (ii) subjecting the mixture of (i) to a selected acid medium under conditions allowing the peptide to exchange with any endogenous peptide for binding with an HLA class I molecule at the surface of said membrane vesicle, (iii) neutralizing the medium to stop the exchange and stabilize the complex formed in (ii) and, (iv) collecting the loaded membrane vesicle.

15. The method of claim 14, wherein step (ii) comprises subjecting the mixture to a selected acid medium at a pH comprised between about 4 and 5.5. for less than 2 hours.

16. The method of claim 14, wherein step (i) comprises contacting the isolated or purified membrane vesicle with 5 to 500 µg/ml of class I-restricted peptide in the absence of beta2-microglobulin.

17. The method of claim 11, wherein the peptide is selected from a tumor antigen, a viral antigen, a parasite antigen and a bacterial antigen.

18. The method of claim 14, wherein the peptide is selected from a tumor antigen, a viral antigen, a parasite antigen and a bacterial antigen.

19. The method of claim 1, comprising :
a) the culture of a population of antigen-presenting cells under conditions allowing the release of membrane vesicles by antigen-presenting cells,
b) a membrane vesicle enrichment or purification step, and
c) the contacting of the membrane vesicles with a peptide under conditions allowing the peptide to bind an MHC molecule at the surface of said membrane vesicles to produce peptide-loaded membrane vesicles.

20. The method of claim 19, wherein the antigen-presenting cells are dendritic cells.

21. The method of claim 19 or 20, wherein, prior to or after step c), the membrane vesicles are subjected to a selected acid medium.

22. The method of claim 1, comprising :
a. obtaining a population of antigen-presenting cells
b. culturing the population of antigen-presenting cells under conditions allowing the release of membrane vesicles by antigen-presenting cells,
c. a clarification of the culture supernatant,
d. a concentration of the clarified supernatant,
e. a diafiltration of the concentrated supernatant,
f. the isolation of the membrane vesicles using density cushion centrifugation,
g. the contacting of the membrane vesicles with a peptide to produce peptide-loaded membrane vesicles, and
h. a sterile filtration of the vesicle membranes obtained in g.

## Patentansprüche

1. Ein Verfahren zur Zubereitung eines immunogenen Membranvesikels, wobei das Verfahren das Isolieren oder Aufreinigen des Membranvesikels aus einer biologischen Probe und das In-Kontakt-Bringen des aufgereinigten Membranvesikels mit einem Peptid oder einem Lipid unter Bedingungen, die es dem Peptid oder Lipid erlauben, an ein Antigen-präsentierendes Molekül auf der Oberfläche des Membranvesikels zu binden, umfasst.

2. Das Verfahren nach Anspruch 1, wobei die Membranvesikel aus einer biologischen Probe, die Antigen-präsentierende Zellen umfasst, isoliert werden.

3. Das Verfahren nach Anspruch 2, wobei die Membranvesikel aus einer biologischen Probe, die humane dendritische Zellen umfasst, isoliert werden.

4. Das Verfahren nach Anspruch 1, wobei das Verfahren den Schritt umfasst: Aussetzen der isolierten oder aufgereinigten Membranvesikel einem ausgewählten Säuremedium vor, während, oder nach dem In-Kontakt-Bringen der Vesikel mit dem Peptid oder Lipid.

5. Das Verfahren nach Anspruch 1, wobei nach dem In-Kontakt-Bringen die Vesikel einer Dichte-Zentrifugation oder Diafiltration unterworfen werden, um ungebundenes Peptid oder Lipid zu entfernen.

6. Das Verfahren nach Anspruch 1, wobei das Peptid ein Klasse-I-beschränktes Peptid ist.

7. Das Verfahren nach Anspruch 1, wobei das Peptid ein Klasse-II-beschränktes Peptid ist.

8. Das Verfahren nach Anspruch 1, wobei die Vesikel mit einem Peptidgemisch in Kontakt gebracht werden.

9. Das Verfahren nach Anspruch 8, wobei die Vesikel mit einem Peptideluat von Tumorzellen in Kontakt gebracht werden.

10. Das Verfahren nach Anspruch 1, wobei das Antigen-präsentierende Molekül ein CD1 Molekül ist und das Lipid ausgewählt wird aus einem mikrobiellen Lipid, einem mikrobiellen Glykolipid und einem Lipid- oder Glykolipid-Tumorantigen.

11. Das Verfahren nach Anspruch 1, zur Zubereitung eines immunogenen Membranvesikels, wobei das Membranvesikel einen Komplex eines exogenen Klasse-I-Peptids, das an ein HLA-Klasse-I-Molekül gebunden ist, umfasst, und wobei das Verfahren die folgenden Schritte umfasst: (i) Aussetzen eines isolierten oder aufgereinigten Membranvesikels einem ausgewählten Säuremedium, (ii) In-Kontakt-Bringen des isolierten oder aufgereinigten Membranvesikels mit einem Klasse-I-beschränkten Peptid in Anwesenheit von beta2-Microglobulin, unter Bedingungen, die es dem Peptid erlauben, mit einem HLA-Klasse-I-Molekül auf der Oberfläche des Membranvesikels zu komplexieren, und (iii) Sammeln der geladenen Membranvesikel.

12. Das Verfahren nach Anspruch 11, wobei der Schritt (i) das Aussetzen der isolierten oder aufgereinigten Membranvesikel einem Medium mit einem pH zwischen etwa 3 und 5,5 für weniger als 15 Minuten umfasst.

13. Das Verfahren nach Anspruch 11, wobei der Schritt (ii) das In-Kontakt-Bringen der isolierten oder aufgereinigten Membranvesikel mit 0,005 bis 50 µg/ml an Klasse-I-beschränktem Peptid in Anwesenheit von beta2-Microglobulin umfasst.

14. Das Verfahren nach Anspruch 1 zur Zubereitung eines immunogenen Membranvesikels, wobei das Membranvesikel einen Komplex eines exogenen Klasse-I-Peptids gebunden an ein HLA-Klasse-I-Molekül umfasst, und wobei das Verfahren (i) das In-Kontakt-Bringen eines isolierten oder aufgereinigten Membranvesikels mit einem Klasse-I-beschränkten Peptid in Abwesenheit eines beta2-Microglobulins, (ii) das Aussetzen des Gemischs aus (i) einem ausgewählten Säuremedium unter Bedingungen, die es dem Peptid erlauben, mit jedem endogenen Peptid die Bindung an ein HLA-Klasse-I-Molekül auf der Oberfläche des Membranvesikels auszutauschen, (iii) das Neutralisieren des Mediums, um den Austausch abzustoppen und den in (ii) gebildeten Komplex zu stabilisieren und, (iv) das Sammeln der geladenen Membranvesikel, umfasst.

15. Das Verfahren nach Anspruch 14, wobei der Schritt (ii) das Aussetzen des Gemischs einem ausgewählten Säuremedium bei einem pH zwischen etwa 4 und 5,5 für weniger als 2 Stunden umfasst.

16. Das Verfahren nach Anspruch 14, wobei Schritt (i) das In-Kontakt-Bringen des isolierten oder auf gereinigten Membranvesikels mit 5 bis 500 µg/ml an Klasse-I-beschränktem Peptid in Abwesenheit von beta2-Microglobulin umfasst.

17. Das Verfahren nach Anspruch 11, wobei das Peptid ausgewählt wird aus einem Tumorantigen, einem viralen Antigen, einem Parasitenantigen und einem bakteriellen Antigen.

18. Das Verfahren nach Anspruch 14, wobei das Peptid ausgewählt wird aus einem Tumorantigen, einem viralen Antigen, einem Parasitenantigen und einem bakteriellen Antigen.

19. Das Verfahren nach Anspruch 1, wobei das Verfahren die folgenden Schritte umfasst:
a) die Kultivierung einer Population an Antigen-präsentierenden Zellen unter Bedingungen, die die Freisetzung von Membranvesikeln durch Antigen-präsentierende Zellen erlauben,
b) ein Membranvesikelanreicherungs- oder -reinigungsschritt, und
c) das In-Kontakt-Bringen der Membranvesikel mit einem Peptid unter Bedingungen, die es dem Peptid erlauben, an ein MHC-Molekül auf der Oberfläche der Membranvesikel zu binden, um Peptid-beladene Membranvesikel zu produzieren.

20. Das Verfahren nach Anspruch 19, wobei die Antigen-präsentierenden Zellen dentritische Zellen sind.

21. Das Verfahren nach Anspruch 19 oder 20, wobei vor oder nach Schritt c) die Membranvesikel einem ausgewählten Säuremedium ausgesetzt werden.

22. Das Verfahren nach Anspruch 1, wobei das Verfahren die folgenden Schritte umfasst:
a. Erhalten einer Population an Antigen-präsentierenden Zellen,
b. Kultivieren der Population Antigen-präsentierender Zellen unter Bedingungen, die die Freisetzung von Membranvesikeln durch Antigen-präsentierende Zellen erlauben,
c. eine Klärung des Kulturüberstands,
d. eine Konzentrierung des geklärten Überstands,
e. eine Diafiltration des konzentrierten Überstands,
f. die Isolierung der Membranvesikel unter Verwendung von Dichtekissen-Zentrifugation,
g. das In-Kontakt-Bringen der Membranvesikel mit einem Peptid, um Peptid-beladene Membranvesikel zu produzieren, und
h. eine Sterilfiltration der in g. erhaltenen Vesikelmembranen.

## Revendications

1. Méthode de préparation d'une vésicule membranaire immunogène, la méthode comprenant l'isolement ou la purification de la vésicule membranaire à partir d'un échantillon biologique et la mise en contact de ladite vésicule membranaire purifiée avec un peptide ou un lipide dans des conditions permettant au peptide ou au lipide de lier une molécule de présentation d'antigène à la surface de ladite vésicule membranaire.

2. La méthode selon la revendication 1, dans laquelle les vésicules membranaires sont isolées à partir d'un échantillon biologique comprenant des cellules présentatrices d'antigènes.

3. La méthode selon la revendication 2, dans laquelle les vésicules membranaires sont isolées à partir d'un échantillon biologique comprenant des cellules dendritiques humaines.

4. La méthode selon la revendication 1, comprenant l'étape consistant à soumettre les vésicules membranaires isolées ou purifiées à un milieu acide sélectionné avant, pendant ou après la mise en contact desdites vésicules avec ledit peptide ou ledit lipide.

5. La méthode selon la revendication 1, dans laquelle, après la mise en contact, les vésicules sont soumises à une centrifugation de densité ou à une diafiltration pour éliminer les peptides ou lipides non liés.

6. La méthode selon la revendication 1, dans laquelle le peptide est un peptide de classe 1 restreint.

7. La méthode selon la revendication 1, dans laquelle le peptide est un peptide de classe II restreint.

8. La méthode selon la revendication 1, dans laquelle les vésicules sont mises en contact avec un mélange de peptides.

9. La méthode selon la revendication 8, dans laquelle les vésicules sont mises en contact avec un éluat de peptides issus de cellules tumorales.

10. La méthode selon la revendication 1, dans laquelle la molécule présentatrice d'antigènes est une molécule CD1 et le lipide est sélectionné parmi un lipide microbien, un glycolipide microbien et un antigène tumoral lipidique ou glycolipidique.

11. La méthode selon la revendication 1, pour préparer une vésicule membranaire immunogène comprenant un complexe formé d'un peptide de classe 1 exogène lié à une molécule HLA de classe I, la méthode comprenant (i) l'exposition d'une vésicule membranaire isolée ou purifiée à un milieu acide sélectionné, (ii) la mise en contact de ladite vésicule membranaire isolée ou purifiée avec un peptide de classe I restreint en présence de microglobuline beta2, dans des conditions permettant au peptide de se complexer avec une molécule HLA de classe I à la surface de ladite vésicule membranaire, et (iii) la récupération de la vésicule membranaire chargée.

12. La méthode selon la revendication 11, dans laquelle l'étape (i) comprend l'exposition des vésicules membranaires isolées ou purifiées à un milieu à un pH compris entre environ 3 et 5.5 pendant moins de 15 minutes.

13. La méthode selon la revendication 11, dans laquelle l'étape (ii) comprend la mise en contact de la vésicule membranaire isolée ou purifiée avec 0.005 à 50 µg/ml de peptide de classe I restreint en présence de microglobuline beta2.

14. La méthode selon la revendication 1, pour préparer une vésicule membranaire immunogène comprenant un complexe formé d'un peptide de classe I exogène lié à une molécule HLA de classe I, la méthode comprenant (i) la mise en contact d'une vésicule membranaire isolée ou purifiée avec un peptide de classe I restreint en l'absence de microglobuline beta2, (ii) l'exposition du mélange obtenu en (i) à un milieu acide sélectionné dans des conditions permettant au peptide de se substituer à tout peptide endogène pour la liaison avec une molécule HLA de classe I à la surface de ladite vésicule membranaire, (iii) la neutralisation du milieu pour stopper la substitution et stabiliser le complexe formé en (ii) et, (iv) la récupération de la vésicule membranaire chargée.

15. La méthode selon la revendication 14, dans laquelle l'étape (ii) comprend l'exposition du mélange à un milieu acide sélectionné à un pH compris entre environ 4 et 5.5 pendant moins de deux heures.

16. La méthode selon la revendication 14, dans laquelle l'étape (i) comprend la mise en contact de la vésicule membranaire isolée ou purifiée avec 5 à 500 µg/ml de peptide de classe I restreint en l'absence de microglobuline beta2.

17. La méthode selon la revendication 11, dans laquelle le peptide est sélectionné parmi un antigène tumoral, un antigène viral, un antigène parasitaire et un antigène bactérien.

18. La méthode selon la revendication 14, dans laquelle le peptide est sélectionné parmi un antigène tumoral, un antigène viral, un antigène parasitaire et un antigène bactérien.

19. La méthode selon la revendication 1, comprenant :
a) la culture d'une population de cellules présentatrices d'antigènes dans des conditions permettant le relarguage des vésicules membranaires par des cellules présentatrices d'antigènes,
b) une étape d'enrichissement ou de purification des vésicules membranaires, et
c) la mise en contact des vésicules membranaires avec un peptide dans des conditions permettant au peptide de se lier avec une molécule du CMH à la surface desdites vésicules membranaires pour produire des vésicules membranaires chargées en peptides.

20. La méthode selon la revendication 19, dans laquelle les cellules présentatrices d'antigènes sont des cellules dendritiques.

21. La méthode selon la revendication 19 ou 20, dans laquelle, avant ou après l'étape c), les vésicules membranaires sont exposées à un milieu acide sélectionné.

22. La méthode selon la revendication 1, comprenant:
a) l'obtention d'une population de cellules présentatrices d'antigènes
b) la culture de la population de cellules présentatrices d'antigènes dans des conditions permettant le relarguage de vésicules membranaires par les cellules présentatrices d'antigènes,
c) une clarification du surnageant de culture,
d) une concentration du surnageant clarifié,
e) une diafiltration du surnageant concentré,
f) l'isolement des vésicules membranaires en utilisant une centrifugation sur coussin de densité,
g) la mise en contact des vésicules membranaires avec un peptide pour produire des vésicules membranaires chargées en peptides, et
h) une filtration stérile des vésicules membranaires obtenues en g .
